(19) Europäisches Patentamt
European Patent Office
Office européen des brevets

(11) Publication number: **0 226 217 B1**

(12) # EUROPEAN PATENT SPECIFICATION

(45) Date of publication of patent specification: **26.01.94**

(51) Int. Cl.[5]: **C07K 7/06**, C07K 7/30, A61K 37/02

(21) Application number: **86117612.1**

(22) Date of filing: **17.12.86**

The file contains technical information submitted after the application was filed and not included in this specification

(54) **Peptides with sulfate ester group.**

(30) Priority: **19.12.85 US 810948**
**18.11.86 US 932119**

(43) Date of publication of application:
**24.06.87 Bulletin 87/26**

(45) Publication of the grant of the patent:
**26.01.94 Bulletin 94/04**

(84) Designated Contracting States:
**AT BE CH DE ES FR GB GR IT LI LU NL SE**

(56) References cited:
**EP-A- 0 107 860**
**EP-A- 0 161 468**
**US-A- 4 351 829**
**US-A- 4 490 364**

**Chemical Abstracts, vol. 104, no. 25, June 23, 1986, page 648, ref. no. 225218t; Columbus, Ohio, US; & JP-A-60 194 000**

**Chemical Abstracts, vol. 102, no. 19, May 13, 1985, pages 169-170, ref.no. 139403e; Columbus, Ohio, US; G. Stacher et al.: "Ceruletide decreases food intake in nonobese man" & Peptides (Fayetteville, N.Y.) 1982, 3(4), 607-12**

(73) Proprietor: **FISONS CORPORATION (a Massachusetts corporation)**
**Jefferson Road, P.O. Box 1710**
**Rochester, NY 14603(US)**

(72) Inventor: **Rosamond, James Donald**
**187 Burlington Avenue**
**Rochester New York 14617(US)**

(74) Representative: **Wright, Robert Gordon McRae**
**FISONS plc**
**12 Derby Road**
**Loughborough Leicestershire LE11 0BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical Abstracts, vol. 97, no. 17, October 25, 1982, page 96, ref.no. 160854j; Columbus, Ohio, US; I.L. Taylor et al.: "Effects of pancreatic polypeptide, caerulein, and bombesin on satiety in obese mice" & AM. J. Physiol. 1985, 248(3, Pt. 1), G277-G280

Peptides chemistry and Biology Proc. 10th Am. Peptide Symp., St. Louis, May 23-28, 1987, Publ. 1988, pages 610-612; ESCOM, Leiden, NL J.D. ROSAMOND et al.: "Structural requirements for the satiety effect of CCK-8"

## Description

BACKGROUND OF THE INVENTION

This invention concerns sulphate ester containing peptides possessing feeding inhibition properties. These peptides have 6 to 8 amino acids. They all differ structurally, however, from two similarly sized peptides known to have feeding inhibition properties: CCK-8, which has the structure Asp-Tyr($SO_3H$)-Met-Gly-Trp-Met-Asp-Phe-$NH_2$, and ceruletide, which has the structure Glp-Gln-Asp-Tyr($SO_3H$)-Thr-Gly-Trp-Met-Asp-Phe-$NH_2$.

The peptides of this invention are not found in nature but, rather, must be synthesized.

SUMMARY OF THE INVENTION

The compounds of the invention are peptides of the formula (1)

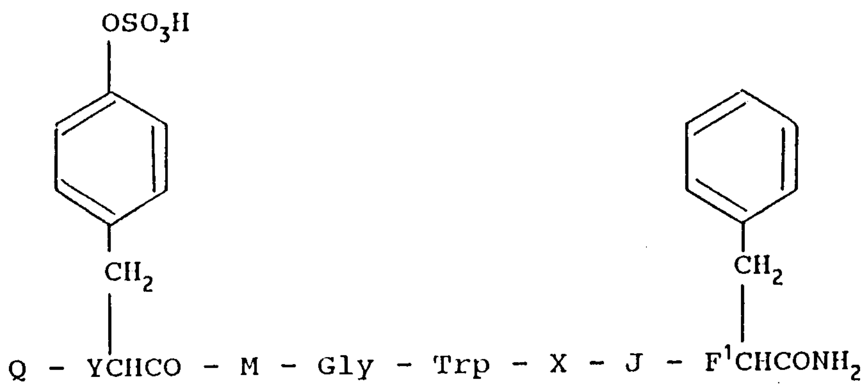

(1)

wherein
Q is H-$\beta$Asp, H-DAsp, For, Ac, Suc, desQ or $R^1R^2$CHOCO;
Y is H, (S)-NH or (S)-$R^3$N;
M is Met, Ahx, Leu or Ile;
X is Met, Ahx, Leu or Ile;
J is Asp, DAsp or MeAsp;
$F^1$ is (S)-NH or (S)-$R^4$N;
$R^1$ and $R^2$ independently represent H or lower alkyl;
$R^3$ and $R^4$ represent lower alkyl;
and pharmaceutically acceptable salts thereof;
provided that:

(1) Q is desQ when Y is H;
(2) Q is not Ac if, in the same peptide: Y is (S)-NH; M is Met, Ahx or Leu; X is Met, Ahx or Leu; J is Asp; and $F^1$ is (S)-NH;
(3) Q is neither H-$\beta$Asp nor For if, in the same peptide: Y is (S)-NH; M is Met, Ahx or Leu; X is Met, Ahx or Leu; J is Asp; and $F^1$ is (S)-NH;
(4) Y is not H if, in the same peptide: M is Met; X is Met; J is Asp; and $F^1$ is (S)-NH;
(5) Q is not Suc if, in the same peptide: Y is (S)-NH; M is Met; X is Met; and J is Asp; and
(6) Q is not H-DAsp if, in the same peptide: Y is (S)-NH; M is Met; X is Met; J is Asp; and $F^1$ is (S)-NH.

The invention is also a process of making the peptides of the invention.

The invention is also methods of treating obesity and preventing obesity, respectively; each such method comprising the administration, by either an intraperitoneal, intravenous, intramuscular, subcutaneous or intranasal route, to a mammal in need of such treatment, of a peptide of the formula (1), without the provisos (2)-(6)
and pharmaceutically acceptable salts thereof.

DETAILED DESCRIPTION

Definitions

"lower alkyl" contains 1 to 6 carbon atoms.

(S) refers to the absolute configuration about the adjacent methine carbon. When Y is (S)-NH, then

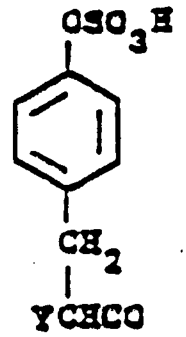

is of the L-configuration.

Similarly, when $F^1$ is (S)-NH then

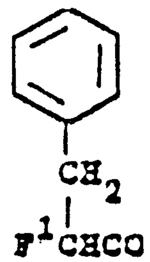

is of the L-configuration.

All optically active amino acids are of the L-configuration unless otherwise indicated.

The H in H-$\beta$Asp and H-DAsp stands for hydrogen.

DesQ, which arises when Y is H, means that there is no Q.

$R^1R^2CHOCO$ is the formula

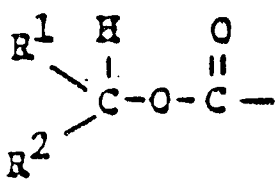

Hpp($SO_3H$) is the formula

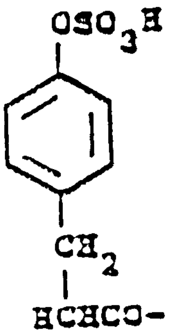

Each claim to a compound includes its pharmaceutically acceptable base addition salts. Base addition salts include those derived from both organic and inorganic bases, such as, for example, ammonia, sodium hydroxide, calcium hydroxide, barium hydroxide, tetraethylammonium hydroxide, ethylamine, diethylamine, triethylamine, and the like.

In an alternative representation to formula (1), used for purposes of brevity, peptides are also represented in accordance with conventional representation, for example,

H-DAsp-Tyr($SO_3H$)-Met-Gly-Trp-Met-Asp-Phe-$NH_2$,

which stands for the compound of formula (1) in which Q is H-DAsp, Y is (S)-NH, X is Met, J is Asp, and $F^1$ is (S)-NH.

When amino acids, peptides, protecting groups, active groups, etc. are represented by symbols in this specification and appended claims, usual symbols as defined by IUPAC and IUB or as used in the art are employed.

Examples of symbols are given below.

| | |
|---|---|
| Abu | 2-aminobutyric acid |
| Ac | acetyl |
| Ahx | 2-aminohexanoic acid |
| Aib | 2-aminoisobutyric acid |
| Ala | alanine |
| Arg | arginine |
| Asn | asparagine |
| Asp | aspartic acid |
| $\beta$Asp | beta-aspartic acid |
| Boc | tert-butyloxycarbonyl |
| $BrCH_2$-Pam | 4-(bromomethyl)phenylacetamidomethyl |
| Cbz | carbobenzoxy |
| Cys(Me) | S-methylcysteine |
| DAla | D-alanine |
| DAhx | D,2-aminohexanoic acid |
| DAsp | D-aspartic acid |
| DMet | D-methionine |
| DPhe | D-phenylalanine |
| DPhe-$NH_2$ | D-phenylalanine amide |
| DTrp | D-tryptophan |
| DTyr | D-tyrosine |
| EtOCO | ethyloxycarbonyl |
| EtPhe | N-ethylphenylalanine |
| EtPhe-$NH_2$ | N-ethylphenylalanine amide |
| Fmoc | 9-fluorenylmethyloxycarbonyl |
| For | formyl |
| Gln | glutamine |
| Glp | pyroglutamyl |
| Glu | glutamic acid |

| | |
|---|---|
| Glt | $HOOC(CH_2)_3CO$- |
| Gly | glycine |
| His | histidine |
| Hpp | 3-(4-hydroxyphenyl)propionyl |
| Hpp($SO_3H$) | 3-(O-sulfo-4-oxyphenyl)propionyl |
| iBuOCO | isobutyloxycarbonyl |
| Ile | isoleucine |
| Leu | leucine |
| Lys | lysine |
| MeAhx | N-methyl-2-aminohexanoic acid |
| MeAsp | N-methylaspartic acid |
| MeLeu | N-methylleucine |
| MeIle | N-methylisoleucine |
| MeMet | N-methylmethionine |
| MeOCO | methyloxycarbonyl |
| MePhe | N-methylphenylalanine |
| MePhe-$NH_2$ | N-methylphenylalanine amide |
| Met | methionine |
| MetO | methionine sulfoxide |
| MeTrp | N-$\alpha$-methyltryptophan |
| MeTyr | N-methyltyrosine |
| MeTyr(Me) | N,O-dimethyltyrosine |
| MeTyr(Me)-$NH_2$ | N,O-dimethyltyrosine amide |
| Mox | methoxinine |
| Nal | 3-(2-naphthyl)alanine |
| OBt | 1-benzotriazolyl ester |
| $OCH_2$-Pam | 4-(oxymethylphenyl)acetamidomethyl |
| OSu | succinimidyloxy ester |
| OtBu | tert-butyl ester |
| Phe | phenylalanine |
| Phe-$NH_2$ | phenylalanine amide |
| Phe-NHEt | phenylalanine ethylamide |
| Phe-NHMe | phenylalanine methylamide |
| Phe-$N(Et)_2$ | phenylalanine diethylamide |
| Phe-$N(Me)_2$ | phenylalanine dimethylamide |
| Phe-OH | phenylalanine acid |
| Phe(4-Cl) | 3-(4-chlorophenyl)alanine |
| Phe(4-Cl)-$NH_2$ | 3-(4-chlorophenyl)alanine amide |
| Phe(4-Me) | 3-(4-methylphenyl)alanine |
| Phe(4-Me)-$NH_2$ | 3-(4-methylphenyl)alanine amide |
| Phe(4-$NO_2$) | 3-(4-nitrophenyl)alanine |
| Phe(4-$NO_2$)-$NH_2$ | 3-(4-nitrophenyl)alanine amide |
| Phe(4-$NH_2$) | 3-(4-aminophenyl)alanine |
| Phe(4-$NH_2$)-$NH_2$ | 3-(4-aminophenyl)alanine amide |
| Pht | 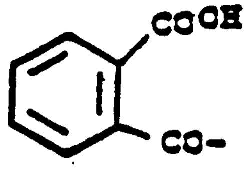 |
| Pro | proline |
| PrOCO | n-propyloxycarbonyl |
| resin | polystyrene |
| Sar | sarcosine |

| | |
|---|---|
| Ser | serine |
| Suc | $HOOC(CH_2)_2CO$- |
| tBu | tert-butyl |
| Thr | threonine |
| Trp | tryptophan |
| Trp(5-F) | 5-fluorotryptophan |
| Trp(6-F) | 6-fluorotryptophan |
| Trp(Me) | 1-methyltryptophan |
| Tyr | tyrosine |
| Tyr-$NH_2$ | tyrosine amide |
| Tyr(Me) | O-methyltyrosine |
| Tyr(Me)-$NH_2$ | O-methyltyrosine amide |
| Tyr($SO_3H$) | O-sulfotyrosine |
| Tyr($SO_3H$)-$NH_2$ | O-sulfotyrosine amide |
| Val | valine |

Preferred Compounds

The preferred compounds from the point of view of feeding inhibition are:

H-DAsp-Tyr($SO_3H$)-Met-Gly-Trp-Met-Asp-Phe-$NH_2$

iBuOCO-Tyr($SO_3H$)-Met-Gly-Trp-Met-Asp-Phe-$NH_2$

Suc-Tyr($SO_3H$)-Ahx-Gly-Trp-Ahx-Asp-Phe-$NH_2$

H-βAsp-Tyr($SO_3H$)-Met-Gly-Trp-Met-Asp-MePhe-$NH_2$

H-DAsp-Tyr($SO_3H$)-Met-Gly-Trp-Met-Asp-MePhe-$NH_2$

For-Tyr($SO_3H$)-Met-Gly-Trp-Met-Asp-MePhe-$NH_2$

iBuOCO-Tyr($SO_3H$)-Met-Gly-Trp-Met-Asp-MePhe-$NH_2$

Hpp($SO_3H$)-Met-Gly-Trp-Met-Asp-MePhe-$NH_2$

PrOCO-Tyr($SO_3H$)-Met-Gly-Trp-Met-Asp-Phe-$NH_2$

EtOCO-Tyr($SO_3H$)-Met-Gly-Trp-Met-Asp-Phe-$NH_2$

MeOCO-Tyr($SO_3H$)-Met-Gly-Trp-Met-Asp-Phe-$NH_2$

H-βAsp-Tyr($SO_3H$)-Met-Gly-Trp-Met-Asp-Phe-$NH_2$

Suc-Tyr($SO_3H$)-Met-Gly-Trp-Met-Asp-Phe-$NH_2$

Hpp($SO_3H$)-Met-Gly-Trp-Met-Asp-Phe-$NH_2$

For-Tyr($SO_3H$)-Met-Gly-Trp-Met-Asp-Phe-$NH_2$

Suc-Tyr($SO_3H$)-Ahx-Gly-Trp-Ahx-Asp-MePhe-$NH_2$

iBuOCO-Tyr($SO_3H$)-Ahx-Gly-Trp-Ahx-Asp-MePhe-$NH_2$

Hpp($SO_3H$)-Ahx-Gly-Trp-Ahx-Asp-MePhe-$NH_2$

The compound, EtOCO-Tyr($SO_3H$)-Met-Gly-Trp-Met-Asp-Phe-$NH_2$, inhibited feeding by 72% during the 0.5 hour feeding period when administered at 0.3 μg/kg in the test described under "Utility" below.

Preparation of Peptides

The novel sulfate ester peptides of this invention and the novel intermediates thereof may be prepared by methods well known to the art, for example, they may be prepared by combining individual amino acids on a solid phase resin on a step-by-step basis, or alternatively, by combining groups of amino acids on a solid phase resin to yield the desired peptidyl-resin intermediate. Such additions, as is known, are accomplished by protecting the amino group of the amino acid or group of amino acids by converting it to, for example, its tert-butyloxycarbonyl (Boc) or 9-fluorenylmethyloxycarbony (Fmoc) derivative, and then activating the carboxylic group of such amino acid or group of amino acids by converting it, for example, to its 1-hydroxyberizotriazole (HOBt) or N-hydroxysuccinimide (HOSu) ester derivative. Such a protected-activated intermediate is then allowed to react with an amino acid-resin or peptidyl-resin with a free amino group, thus extending the peptide chain to provide the peptidyl-resin of formula 2, wherein Pg is a suitable protecting group, for example, Boc or Fmoc.

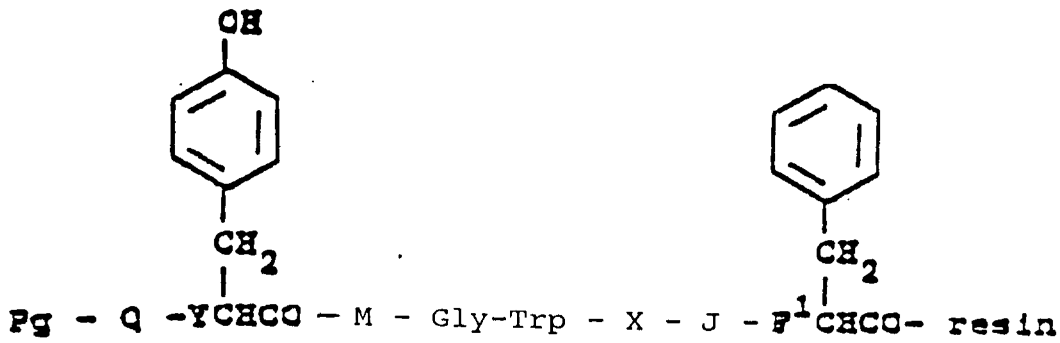

(2)

The phenolic OH group within formula (2) is converted to a sulfate ester by the use of a usual sulfating agent, such as sulfur trioxide pyridine complex. More specifically, the reaction is conducted, for example, by suspending a peptidyl-resin of formula 2 in dimethylformamide (DMF), pyridine or like solvent, and adding sulfur trioxide pyridine complex in about 10-40 molar excess to provide the sulfated peptidyl-resin of the formula 3.

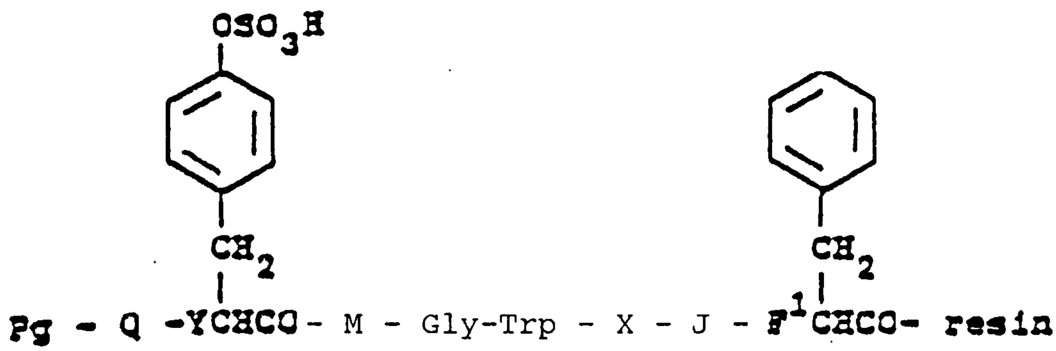

(3)

Since the sulfate ester containing peptide end-products of this invention are C-terminal amides, the chemical link which connects the peptide chain to the resin must be such that its cleavage with suitable reagents readily provides amides. Due to the lability of the sulfate ester group to strong acids (for example, liquid hydrogen fluoride), the peptidyl-resin linkage may be cleavable with either weaker acids (for example, brief treatment with trifluoroacetic acid, TFA) and/or nucleophiles (for example, ammonia, amines, hydroxide, and alkoxides). Among the suitable resin derivatives may be mentioned oxymethyl-polystyrene, 4-(oxymethyl-phenyl)$(CH_2)_n$CO-aminomethyl-polystyrene (n = 0-3) and 4-(oxymethylphenyl)oxymethyl-polystyrene. Similarly substituted polyacrylamide resins are equally well suited as the above polystyrene based resins. For the purposes of this invention the 4-(oxymethylphenyl)$CH_2$CO-aminomethyl-polystyrene [herein referred to as 4-(oxymethylphenyl)acetamidomethylpolystyrene or $OCH_2$-Pam-resin] is best suited for the generation of peptide amides. Thus, this invention describes a process for the synthesis of sulfated peptidyl-$OCH_2$-Pam-resins of formula (4), wherein the resin is polystyrene (the term "polystyrene" includes copolymers with minor amounts, usually 1%, of unsaturated monomers such as divinylbenzene).

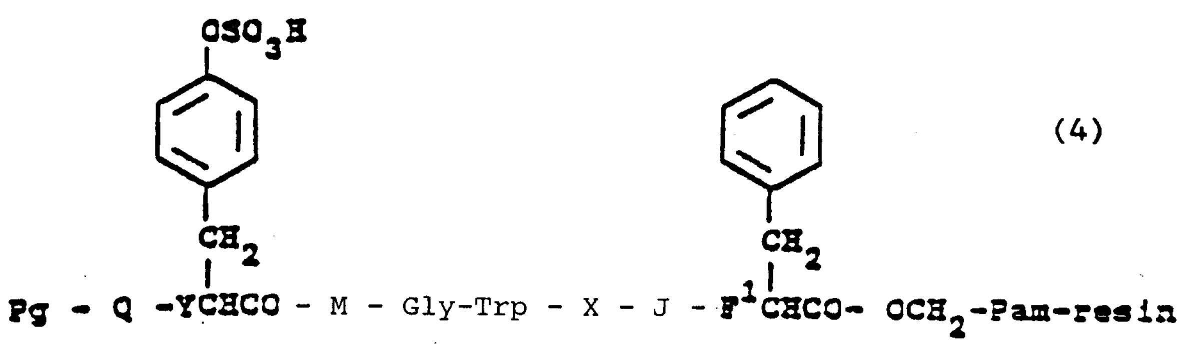

(4)

In forming peptide sequences of this invention, the amino functions may be protected by commonly used amino protecting groups such as Boc, Fmoc, (4-methoxybenzyl)oxycarbonyl, 2-nitrophenylsulfenyl, and so forth. The Boc and Fmoc protecting groups are preferred. The carboxyl and hydroxyl protecting group may be methyl, tert-butyl (tBu), benzyl, 4-methoxybenzyl and so forth. The tBu group is preferred.

The amino acid defined by the $F^1$ group of formula 4 may be attached to the $OCH_2$-Pam-resin in several ways. (a) For example, Boc protected-phenylalanine, wherein $F^1$ is (S)-NH may be reacted with a suitable 4-(bromomethyl)-phenylacetate ester (for example, phenacyl ester) and processed further to provide Boc-Phe-(4-oxymethylphenyl)acetic acid which may be coupled to aminomethyl-polystyrene to provide Boc-Phe-(4-oxymethylphenyl)acetamidomethylpolystyrene (Boc-Phe-$OCH_2$-Pam-resin). (b) Alternatively, 4-(bromomethyl)phenylacetic acid may be coupled to aminomethylpolystyrene to provide 4-(bromomethyl)phenylacetamidomethylpolystyrene ($BrCH_2$-Pam-resin) which may be reacted with the cesium salt of Boc-Phe-OH to provide Boc-Phe-$OCH_2$-Pam-resin.

Among the suitable activating groups may be mentioned any combination of groups which causes the acid function of the amino acid to become more reactive, such as acid chlorides, mixed and symmetrical anhydrides, reaction product with carbodiimide (for example, dicyclohexylcarbodiimide, DCC), and active esters (for example, esters derived from HOBt, HOSu, 2- or 4-nitrophenol, and 2,4,5-trichlorophenol). The use of DCC and esters of HOBt and HOSu is particularly preferred from the standpoint of yield, lack of by-products, and consequent ease of purification.

The protecting groups are removed by known reactions such as treatment with dilute TFA (50% in dichloromethane, DCM) for Boc and/or tBu removal and treatment with dilute piperidine (20% in DMF) for Fmoc removal, to name a few, to provide the sulfated peptidyl-resin of the formula (5).

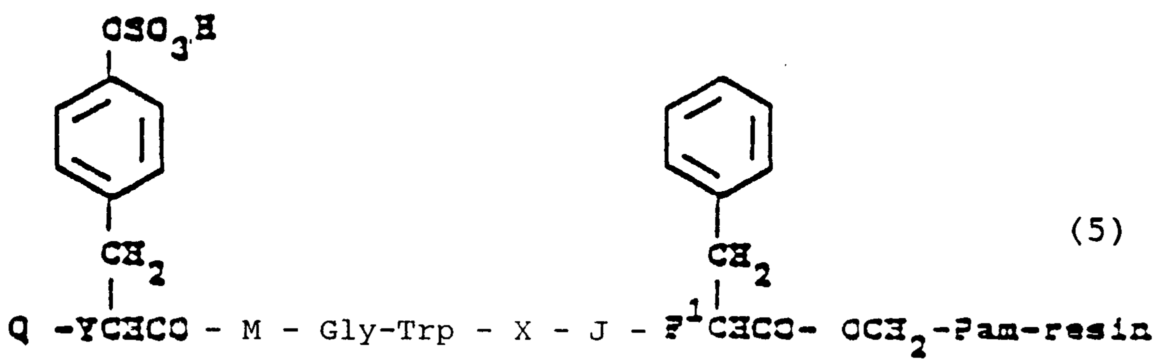

(5)

The sulfate ester containing peptides of formula 1 may be obtained by cleavage of the peptidyl-$OCH_2$-Pam-resin linkage of (5) with the appropriate reagent. The C-terminal sulfated peptide amides are derived, for example, by treatment of the sulfated peptidyl-resin of formula (5) with methanolic solutions of ammonia.

An automatic peptide synthesizer was used for the solid phase synthesis of the sulfated peptide amides of this invention. The protocol of coupling onto aminomethyl-resin or peptidyl-$OCH_2$-Pam-resin (1 mmole of available nitrogen), deprotection, sulfation, cleavage, and product purification is set forth in Table 1.

Table 1. Protocol for solid phase synthesis of sulfated peptide amides (1 mmole scale). Each step volume is 50 ml unless otherwise indicated. All wash steps are repeated three times. Abbreviations: DCC, dicyclohexylcarbodiimide; DCM, dichloromethane; DIEA, N,N-diisopropylethylamine, DMF, dimethylformamide; HOBt, 1-hydroxybenzotriazole; TFA, trifluoroacetic acid.

| Step | Reagent or Solvent | Purpose | Mix Time |
|---|---|---|---|
| 1 | DCM | Wash | 1 min |
| 2 | Go to Step 3, 5, or 8 | - - - | - - - |
| 3 | Add filtered, pre-activated (0°C, 1 hr) mixture of protected amino acid (or protected dipeptide, 3 mmole), HOBt (4.5 mmole), and DCC (3 mmole) in 1:4 DMF/DCM | Pre-activated DCC/HOBt coupling | 2-15 hr |
| 4 | Go to Step 10, 16, 21, or 26 | - - - | - - - |
| 5 | Add protected amino acid (or protected dipeptide, 3 mmole) and HOBt (4.5 mmole) in 30 ml 1:2 DMF/DCM then DCC (3 mmole) in 20 ml DCM | In situ activated DCC/HOBt coupling | 2-15 hr |
| 6 | 2-Propanol | Wash | 1 min |
| 7 | Go to Step 4 | - - - | - - - |
| 8 | Add active ester or anhydride (3 mmole) in DCM, DMF, or mixture thereof | Non DCC/HOBt activated coupling | 2-15 hr |
| 9 | Go to Step 4 | - - - | - - - |
| 10 | DCM | Wash | 1 min |
| 11 | Treat with 49:1:50 TFA/anisole/DCM | Boc and tBu removal | 30 min |
| 12 | DCM | Wash | 1 min |
| 13 | Treat with 1:19 DIEA/DCM | Neutralize | 1 min |
| 14 | DCM | Wash | 1 min |
| 15 | Go to Step 1, 16, 21, or 26 | - - - | - - - |
| 16 | DMF | Wash | 1 min |
| 17 | Treat with 1:4 piperidine/DMF | Fmoc removal | 3 min |
| 18 | Treat with 1:4 piperidine/DMF | Fmoc removal | 7 min |
| 19 | DMF | Wash | 1 min |
| 20 | Go to Step 15 | - - - | - - - |
| 21 | DMF | Wash | 1 min |
| 22 | 1:2 pyridine/DMF | Wash | 1 min |
| 23 | Add sulfur trioxide pyridine complex (40 mmole) in 60 ml 1:2 pyridine/DMF | Sulfation | 20-24 hr |
| 24 | DMF | Wash | 1 min |
| 25 | Go to Step 4 | - - - | - - - |
| 26 | Methanol | Wash | 1 min |
| 27 | Ammonia saturated (-20°C) methanol or 20% methanolic amine (250 ml) | Resin cleavage | 2-5 day |
| 28 | Methanol | Wash | 1 min |
| 29 | Combine, concentrate filtrates from Steps 27-28 | Isolation | - - - |
| 30 | Chromatograph residue on column(s) of Amberlite XAD-2 (Rohm and Haas, 2.5 x 60 cm, methanol gradient 0.1 M in ammonia), Trisacryl M DEAE (LKB Inc., 2.5 x 47 cm, ammonium bicarbonate gradient), and/or P-40 ODS-3 (Whatman, 4.8 x 50 cm, methanol gradient 0.2% in ammonium acetate) | Purification | - - - |

The sulfate ester containing peptides of formula (1) thus prepared may be desalted and purified by the usual methods. For example, the product may be purified by ion-exchange chromatography with use of Trisacryl M DEAE, DEAE-cellulose or the like, partition chromatography with use of Sephadex LH-20, Sephadex G-25 or the like, reverse phase chromatography with use of Amberlite XAD-2, ODS-silica gel or the like, normal phase chromatography with use of silica gel or the like, or high-performance liquid chromatography (HPLC).

Analogous procedures, wherein the reactions are carried out without the solid phase component (resin), are well known in the art and well suited to large scale production. [See, e.g., U.S. Patent 3,892,726.]

Utility

The peptides of this invention have the ability to inhibit feeding activity in mammals. As a result they have utility in the prevention and treatment of obesity. Feeding inhibition activity can be demonstrated in rats as follows:

Male Sprague-Dawley rats (weighing 300-350 g) are individually caged and maintained on a 12 hr light, dark cycle and trained for at least 14 days to feed during a three hr period of the dark cycle but not the 21 hours preceding that three hr period. The day of the study, rats are dosed intraperitoneally with saline (controls) or test compound (dissolved in saline; usually at a concentration of 0.3 to 300 micrograms of test compound per kg of rat weight). Food is introduced 10 minutes after administration of saline or test compound. Test compounds are deemed to be active if the test group consumes significantly less food than the saline controls during the feeding period which ends either 0.5 or 3 hr after presentation of food. The % feeding inhibition for the 0.5 hr feeding period obtained by administering a dose of 30 μg/kg of test compound is given in Table 2 for CCK-8 and various compounds of the invention.

TABLE 2

| STRUCTURE | % FEEDING INHIBITION |
|---|---|
| H-Asp-Tyr($SO_3H$)-Met-Gly-Trp-Met-Asp-Phe-$NH_2$ | 70 |
| H-DAsp-Tyr($SO_3H$)-Met-Gly-Trp-Met-Asp-Phe-$NH_2$ | 99 |
| iBuOCO-Tyr($SO_3H$)-Met-Gly-Trp-Met-Asp-Phe-$NH_2$ | 100 |
| Suc-Tyr($SO_3H$)-Ahx-Gly-Trp-Ahx-Asp-Phe-$NH_2$ | 100 |
| H-βAsp-Tyr($SO_3H$)-Met-Gly-Trp-Met-Asp-MePhe-$NH_2$ | 100 |
| H-DAsp-Tyr($SO_3H$)-Met-Gly-Trp-Met-Asp-MePhe-$NH_2$ | 100 |
| For-Tyr($SO_3H$)-Met-Gly-Trp-Met-Asp-MePhe-$NH_2$ | 100 |
| iBuOCO-Tyr($SO_3H$)-Met-Gly-Trp-Met-Asp-MePhe-$NH_2$ | 100 |
| Hpp($SO_3H$)-Met-Gly-Trp-Met-Asp-MePhe-$NH_2$ | 100 |
| PrOCO-Tyr($SO_3H$)-Met-Gly-Trp-Met-Asp-Phe-$NH_2$ | 90 |
| EtOCO-Tyr($SO_3H$)-Met-Gly-Trp-Met-Asp-Phe-$NH_2$ | 89 |

| | |
|---|---|
| MeOCO-Tyr($SO_3H$)-Met-Gly-Trp-Met-Asp-Phe-$NH_2$ | 100 |
| H-βAsp-Tyr($SO_3H$)-Met-Gly-Trp-Met-Asp-Phe-$NH_2$ | 91 |
| Suc-Tyr($SO_3H$)-Met-Gly-Trp-Met-Asp-Phe-$NH_2$ | 75 |
| Hpp($SO_3H$)-Met-Gly-Trp-Met-Asp-Phe-$NH_2$ | 99 |
| For-Tyr($SO_3H$)-Met-Gly-Trp-Met-Asp-Phe-$NH_2$ | 100 |
| Suc-Tyr($SO_3H$)-Ahx-Gly-Trp-Ahx-Asp-MePhe-$NH_2$ | 100 |
| iBuOCO-Tyr($SO_3H$)-Ahx-Gly-Trp-Ahx-Asp-MePhe-$NH_2$ | 100 |
| Hpp($SO_3H$)-Ahx-Gly-Trp-Ahx-Asp-MePhe-$NH_2$ | 94 |
| iBuOCO-Tyr($SO_3H$)-Ahx-Gly-Trp-Ahx-Asp-Phe-$NH_2$ | NT |
| Hpp($SO_3H$)-Ahx-Gly-Trp-Ahx-Asp-Phe-$NH_2$ | NT |
| For-Tyr($SO_3H$)-Ile-Gly-Trp-Ile-Asp-Phe-$NH_2$ | NT |
| Suc-Tyr($SO_3H$)-Ile-Gly-Trp-Ile-Asp-Phe-$NH_2$ | 73 |
| iBuOCO-Tyr($SO_3H$)-Ile-Gly-Trp-Ile-Asp-Phe-$NH_2$ | 62 |
| Hpp($SO_3H$)-Ile-Gly-Trp-Ile-Asp-Phe-$NH_2$ | 87 |

(END OF TABLE 2)

NT means "not tested"

An appropriate procedure for administering a peptide designated here as suitable for intraperitoneal, intravenous, intramuscular, subcutaneous, or intranasal administration, to a mammal in need of either treatment for obesity or prevention of obesity, is at a dose of about 0.3 micrograms (μg) to 3 mg per kg of body weight per day, either as single dose or divided among two to four doses. The dosage may be varied, depending upon the requirements of the patient and the compound being employed.

EXAMPLES

The invention may be further illustrated by the following examples. Examples 1-18 illustrate the synthesis of intermediates. Examples 19- 42 illustrate the synthesis of compounds that have been designated above as either a compound of the invention or a compound useful in the treatment and prevention of obesity. The examples are intended to illustrate the invention, not to limit it in any manner.

Peptide syntheses, unless otherwise stated, were initiated with 1 milliequivalent of aminomethyl resin, where the resin was 99:1 by weight styrene:divinylbenzene copolymer. Reactions were performed at room temperature unless otherwise stated.

Washing steps were performed three times with 50 ml of the specified solvent unless otherwise stated.

EXAMPLE 1

Isobutyl Succinimidyl Carbonate (iBuOCO-OSu) To a solution of isobutyl chloroformate (26 ml, 200 mmole) in 600 ml of chloroform was added in portions the dicyclohexylamine (DCHA) salt of N-hydroxysuccinimide (HOSu, 49.28 g, 200 mmole). After stirring the resulting suspension overnight, the precipitated DCHA hydrochloride was filtered off and washed with chloroform. The concentrated filtrate (ca. 50 ml) and washings were diluted with 400 ml of ethyl acetate (EtOAc) and washed with 10% citric acid (4 x 100 ml), brine (2 x 100 ml), 10% sodium bicarbonate (3 x 100 ml), and brine (4 x 100 ml) and then dried (magnesium sulfate), filtered, and concentrated to ca. 100 ml. On diluting with ether and precipitating with hexane, 26.6 g (62% yield) of iBuOCO-OSu was obtained, mp 33-35 °C.

EXAMPLE 2

Fmoc-DTyr-OH

D-Tyrosine (1.81 g) was dissolved in 20 ml of water and 30 ml of tetrahydrofuran (THF) with 10 ml of N sodium hydroxide. Solid 9-fluorenylmethyl succinimidyl carbonate (Fmoc-OSu, 3.37 g) was added with rapid stirring. The suspension was adjusted to pH 7 with N sodium hydroxide and stirred overnight. Solid citric acid (3 g) was added followed by 60 ml of EtOAc. The EtOAc layer was collected, washed with 10% citric acid, brine, and dried (magnesium sulfate). Evaporation of the EtOAc solution gave a light tan syrup which was crystallized from dichloromethane (DCM) to give 3.9 g of Fmoc-DTyr-OH, mp 178-181 °C.

EXAMPLE 3

Fmoc-Tyr-OH

Following the procedure of Example 2 but substituting L-tyrosine (9.06 g) for D-tyrosine, 18.4 g of Fmoc-Tyr-OH was obtained, mp 172-177 °C.

EXAMPLE 4

Fmoc-MeTyr-OH

Following the procedure of Example 2 but substituting N-methyltyrosine (1.95 g) for D-tyrosine, 1.22 g of Fmoc-MeTyr-OH was obtained, mp 152-158 °C.

EXAMPLE 5

Boc-MePhe-(4-oxymethylphenyl)acetic Acid

To a solution of Boc-MePhe-OH (27.93 g) and 4-(bromomethyl) phenylacetic acid phenacyl ester (33.32 g) in 1000 ml of acetonitrile was added potassium fluoride dihydrate (18.28 g). The suspension was stirred overnight, filtered and the filtrate evaporated to dryness. The residue, Boc-MePhe-(4-oxymethylphenyl)-acetic acid phenacyl ester, was dissolved in 85% acetic acid (1200 ml), treated with zinc dust (128 g), and stirred for 2-4 hrs. Concentration of the filtered reaction mixture to ca. 400 ml and dilution with ca. 3200 ml of water gave an oil which was dissolved in EtOAc and treated with DCHA to give 41.31 g of the DCHA salt of title compound, mp 120-122 °C.

EXAMPLE 6

Boc-EtPhe-(4-oxymethylphenyl)acetic Acid Following the procedure of Example 5 but substituting Boc-EtPhe-OH (7.33 g) for Boc-MePhe-OH, 5.69 g of the DCHA salt of Boc-EtPhe-(4-oxymethylphenyl)acetic acid was obtained, mp 137-141 °C.

EXAMPLE 7

Boc-Phe(4-Cl)-(4-oxymethylphenyl)acetic Acid Following the procedure of Example 5 but substituting Boc-Phe(4-Cl)-OH (2.5 g) for Boc-MePhe-OH, 3.44 g of the free base of Boc-Phe(4-Cl)-(4-oxymethylphenyl)-

acetic acid was obtained.

EXAMPLE 8

Boc-Tyr(Me)-(4-oxymethylphenyl)acetic Acid Following the procedure of Example 5 but substituting Boc-Tyr(Me)-OH (2.5 g) for Boc-MePhe-OH, 1.83 g of the free base of Boc-Tyr(Me)-(4-oxymethylphenyl)-acetic acid was obtained, mp 64-67 °C.

EXAMPLE 9

Fmoc-Tyr(tBu)-(4-oxymethylphenyl)acetic Acid Following the procedure of Example 5 but substituting Fmoc-Tyr(tBu)-OH (6.86 g) for Boc-MePhe-OH, 4.88 g of the free base of Fmoc-Tyr(tBu)-(4-oxymethyl-phenyl)acetic acid was obtained, mp 192-195 °C.

EXAMPLE 10

Fmoc-Met-Asp(OtBu)-OH

Fmoc-Met-OSu was prepared, in situ, by the reaction of Fmoc-Met-OH (14.87 g), HOSu (5.52 g), and dicyclohexylcarbodiimide (DCC, 8.26 g) in THF (200 ml) at 0 °C for 3.5 hrs. Precipitated dicyclohexylurea (DCU) was removed by filtration and the THF filtrate was added to a cold solution of H-Asp (OtBu)-OH in 220 ml of 10:1 water/THF to which had been added 40 ml of N sodium hydroxide. After stirring the reaction mixture at room temperature overnight, solid citric acid (20 g) was added along with EtOAc (600 ml). The EtOAc layer was separated, washed with 10% citric acid and brine, and dried (magnesium sulfate). Evaporation of the EtOAc solution gave a residue which was dissolved in 200 ml of EtOAc and treated with DCHA (7.84 ml) to precipitate 17.93 g of the DCHA salt of the desired product, mp 159-162 °C.

EXAMPLE 11

H-Phe-$OCH_2$-Pam-resin

Boc-Phe-(4-oxymethylphenyl)acetic acid (0.83 g, 2 mmole), 1-hydroxybenzotriazole (HOBt, 0.46 g, 3 mmole) and DCC (0.41 g, 2 mmole) were dissolved in 50 ml of 4:1 DCM/DMF and stirred at 0 °C for 1 hr. Aminomethyl-resin (1.34 g, 1 mmole available nitrogen (was suspended in the filtered reaction mixture (precipitated DCU removed) and shaken for 2 to 15 hours. The product, Boc-Phe-$OCH_2$-Pam-resin, was isolated by filtration and treated according to Table 1 (Steps 10-14) to give the desired free base, H-Phe-$OCH_2$-Pam-resin.

EXAMPLE 12

H-MePhe-$OCH_2$-Pam-resin

Boc-MePhe-(4-oxymethylphenyl)acetic acid (from 1.82 g, 3 mmole, of its DCHA salt, Example 5) and HOBt (0.69 g, 4.5 mmole) in 40 ml of 1:3 DMF/DCM followed by DCC (0.62 g, 3 mmole) in 20 ml of DCM were added to aminomethyl-resin (1.34 g, 1 mmole available nitrogen) to give a suspension which was shaken for 2 to 15 hours. The desired product, Boc-MePhe-$OCH_2$-Pam-resin, was isolated by filtration, washed with 2-propanol and DCM, and treated according to Table 1 (Steps 10-14) to give the desired free base, H-MePhe-$OCH_2$-Pam-resin.

EXAMPLE 13

H-EtPhe-$OCH_2$-Pam-resin

Following the procedure of Example 12 but substituting Boc-EtPhe-(4-oxymethylphenyl)acetic acid (from 1.87 g, 3 mmole, of its DCHA salt, Example 6) for Boc-MePhe-(4-oxymethylphenyl)acetic acid, H-EtPhe-$OCH_2$-Pam-resin was obtained.

EXAMPLE 14

H-Phe(4-Cl)-$OCH_2$-Pam-resin

Following the procedure of Example 11 but substituting Boc-Phe(4-Cl)-(4-oxymethylphenyl)acetic acid (0.90 g, 2 mmole, Example 7) for Boc-Phe-(4-oxymethylphenyl)acetic acid, H-Phe(4-Cl)-$OCH_2$-Pam-resin was obtained.

EXAMPLE 15

H-Phe(4-$NO_2$)-$OCH_2$-Pam-resin

Boc-Phe(4-$NO_2$)-OH (1.39 g) was dissolved in 100 ml of 70% methanol (MeOH) and adjusted to pH 7 with the addition of N cesium bicarbonate. The solution was evaporated to dryness with the residue being evaporated three more times with added DMF. The resultant dried cesium salt of Boc-Phe(4-$NO_2$)-OH was dissolved in 60 ml of DMF and shaken with $BrCH_2$-Pam-resin (1 meq or Br) overnight. The desired product, Boc-Phe(4-$NO_2$)-$OCH_2$-Pam-resin, was isolated by filtration, washed with DCM, and treated according to Table 1 (Steps 10-14) to give the desired free base, H-Phe(4-$NO_2$)-$OCH_2$-Pam-resin.

EXAMPLE 16

H-Tyr(Me)-$OCH_2$Pam-resin

Following the procedure of Example 11 but substituting Boc-Tyr(Me)-(4-oxymethylphenyl)acetic acid (0.87 g, 2 mmole, Example 8) for Boc-Phe-(4-oxymethylphenyl)acetic acid, H-Tyr(Me)-$OCH_2$-Pam-resin was obtained.

EXAMPLE 17

H-Tyr(tBu)-$OCH_2$-Pam-resin

Fmoc-Tyr(tBu)-(4-oxymethylphenyl)acetic acid (1.82 g, 3 mmole, Example 9), 1-hydroxybenzotriazole (HOBt, 0.69 g, 4.5 mmole), and DCC (0.62 g, 3 mmole) were dissolved in 50 ml of 4:1 DCM/DMF and stirred at 0 °C for 1 hr. Aminomethylresin (1.34 g, 1 mmole available nitrogen) was suspended in the filtered reaction mixture (precipitated DCU removed) and shaken for 2 to 15 hours. The product, Fmoc-Tyr(tBu)-$OCH_2$-Pam-resin, was isolated by filtration and treated according to Table 1 (Steps 16-20) to give the desired free base, H-Tyr (tBu)-$OCH_2$-Pam-resin.

EXAMPLE 18

H-MeTyr(Me)-$OCH_2$-Pam-resin

Following the procedure of Example 15 but substituting Boc-MeTyr(Me)-OH (from 1.47 g of its DCHA salt) for Boc-Phe($NO_2$)-OH, H-MeTyr(Me)-$OCH_2$-Pam-resin was obtained.

EXAMPLE 19

H-DAsp-Tyr($SO_3$H)-Met-Gly-Trp-Met-Asp-Phe-$NH_2$

H-Phe-$OCH_2$-Pam-resin (Example 11) (JP-A-60-194 000), was sequentially coupled with Fmoc-Asp(OtBu)-OH, Fmoc-Met-OH, Fmoc-Trp-OH, Fmoc-Gly-OH, Fmoc-Met-OH, Fmoc-Tyr-OH (Example 3), and Fmoc-DAsp(OtBu)-OH according to Table 1 (coupling Steps 3-4 followed by Fmoc removal Steps 16-20) to provide Fmoc-DAsp(OtBu)-Tyr-Met-Gly-Trp-Met-Asp(OtBu)-Phe-$OCH_2$-Pam-resin which was sulfated, deprotected, and cleaved from the resin according to Table 1 (Steps 21-25, Steps 10-20, and then Steps 26-29 with ammonia) to give the title compound which was chromatographically purified on Trisacryl M DEAE according to Table 1 (Step 30) to give 198 mg of the ammonium salt of the title compound. Amino acid analysis following acid decomposition gave Asp 2.11 (2), Tyr 1.04 (1), Met 2.07 (2), Gly 1.08 (1), and Phe 1.04 (1). Infrared absorption spectrum showed a strong peak typical of a sulfuric acid ester at 1050 $cm^{-1}$.

TLC $R_f$ 0.35 (TLC herein refers to chromatography of the title compound on Merck silica gel thin layer plates in the solvent system chloroform-methanol-acetic acid-water, 6:3:1:1.

EXAMPLE 20

iBuOCO-Tyr($SO_3H$)-Met-Gly-Trp-Met-Asp-Phe-$NH_2$

H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, and Fmoc-Tyr(tBu)-OH according to Table 1 (coupling Steps 5-7 followed by Fmoc removal Steps 16-20) to give H-Tyr(tBu)-Met-Gly-Trp-Met-Asp(OtBu)-Phe-$OCH_2$-Pam-resin which was coupled with iBuOCO-OSu (Example 1) according to Table 1 (Steps 8-9) to give iBuOCO-Tyr(tBu)-Met-Gly-Trp-Met-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2, Trisacryl M DEAE, and P-40 ODS-3, sequentially, according to Table 1 (Step 30) to give 206 mg of the ammonium salt of the title compound. Amino acid analysis following acid decomposition gave Asp 1.06 (1), Tyr 1.04 (1), Met 2.04 (2), Gly 1.06 (1), and Phe 1.04 (1). Infrared absorption spectrum showed a strong peak typical of a sulfuric acid ester at 1050 $cm^{-1}$. TLC $R_f$ 0.56.

EXAMPLE 21

Suc-Tyr($SO_3H$)-Ahx-Gly-Try-Ahx-Asp-Phe-$NH_2$

H-Phe-$OCH_2$-Pam-resin (Example 11) was sequentially coupled with Fmoc-Asp(OtBu)-OH, Fmoc-Ahx-OH, Fmoc-Trp-OH, Fmoc-Gly-OH, Fmoc-Ahx-OH, and Fmoc-Tyr(tBu)-OH according to Table 1 (coupling Steps 5-7 followed by Fmoc removal Steps 16-20) to give H-Tyr(tBu)-Ahx-Gly-Trp-Ahx-Asp(OtBu)-Phe-$OCH_2$-Pam-resin which was coupled with succinic anhydride (0.6 g, 6 mmole, in DMF) according to Table 1 (Steps 8-9) to give Suc-Tyr(tBu)-Ahx-Gly-Trp-Ahx-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2, Trisacryl M DEAE, and P-40 ODS-3, sequentially, according to Table 1 (Step 30) to give 240 mg of the ammonium salt of the title compound. Amino acid analysis following acid decomposition gave Asp 1.01 (1), Tyr 0.95 (1), Ahx 2.10 (2), Gly 1.06 (1), and Phe 0.88 (1). Infrared absorption spectrum showed a strong peak typical of a sulfuric acid ester at 1050 $cm^{-1}$. TLC $R_f$ 0.36.

EXAMPLE 22

H-$\beta$Asp-Tyr($SO_3H$)-Met-Gly-Trp-Met-Asp-MePhe-$NH_2$

H-MePhe-$OCH_2$-Pam-resin (Example 12) was sequentially coupled with Fmoc-Met-Asp(OtBu)-OH (Example 10), Fmoc-Trp-OH, Fmoc-Gly-OH, Fmoc-Met-OH, Fmoc-Tyr(tBu)-OH, and Boc-$\beta$Asp(OtBu)-OH according to Table 1 (coupling Steps 5-7 followed by Fmoc removal Steps 16-20) to give Boc-$\beta$Asp(OtBu)-Tyr(tBu)-Met-Gly-Trp-Met-Asp(OtBu)-MePhe-$OCH_2$-Pam-resin which was deprotected according to Table 1 (Steps 10-15) and coupled with Fmoc-OSu (1.1 g) according to Table 1 (Steps 8-9) to give Fmoc-$\beta$Asp-Tyr-Met-Gly-Trp-Met-Asp-MePhe-OCH2-Pam-resin which was sulfated deprotected, and cleaved from the resin according to Table 1 (Steps 21-25, Steps 16-20, and then Steps 26-29 with ammonia) to give the title compound which was chromatographically purified on Amberlite XAD-2, Trisacryl M DEAE, and P-40 ODS-3, sequentially, according to Table 1 (Step 30) to give 72 mg of the ammonium salt of the title compound. Amino acid analysis following acid decomposition gave Asp 2.03 (2), Tyr 1.05 (1), Met 1.85 (2), and Gly 1.10 (1). Infrared absorption spectrum showed a strong peak typical of a sulfuric acid ester at 1050 $cm^{-1}$. TLC $R_f$ 0.33.

EXAMPLE 23

H-DAsp-Tyr($SO_3H$)-Met-Gly-Trp-Met-Asp-MePhe-$NH_2$

H-MePhe-$OCH_2$-Pam-resin (Example 12) was sequentially coupled with Fmoc-Met-Asp(OtBu)-OH (Example 10), Fmoc-Trp-OH, Fmoc-Gly-OH, Fmoc-Met-OH, Fmoc-Tyr(tBu)-OH, and Fmoc-DAsp(OtBu)-OH according

to Table 1 (coupling Steps 3-4 followed by Fmoc removal Steps 16-20) to give Fmoc-DAsp(OtBu)-Tyr(tBu)-Met-Gly-Trp-Met-Asp(OtBu)-MePhe-$OCH_2$-Pam-resin which was deprotected, sulfated, deprotected, and cleaved from the resin according to Table 1 (Steps 10-15, Steps 21-25, Steps 16-20, and then Steps 26-29 with ammonia) to give the title compound which was chromatographically purified on Trisacryl M DEAE and P-40 ODS-3, sequentially, according to Table 1 (Step 30) to give 110 mg of the ammonium salt of the title compound. Amino acid analysis following acid decomposition gave Asp 2.01 (2), Tyr 1.01 (1), Met 1.91 (2), and Gly 1.07 (1). Infrared absorption spectrum showed a strong peak typical of a sulfuric acid ester at 1050 $cm^{-1}$. TLC $R_f$ 0.35.

EXAMPLE 24

For-Tyr($SO_3H$)-Met-Gly-Trp-Met-Asp-MePhe-$NH_2$

H-MePhe-$OCH_2$-Pam-resin (Example 12) was sequentially coupled with Fmoc-Met-Asp(OtBu)-OH (Example 10), Fmoc-Trp-OH, Fmoc-Gly-OH, and Fmoc-Met-OH according to Table 1 (coupling Steps 5-7 followed by Fmoc removal Steps 16-20) to give Fmoc-Met-Gly-Trp-Met-Asp(OtBu)-MePhe-$OCH_2$-Pam-resin which was deprotected according to Table 1 (Steps 10-20) and coupled with For-Tyr-OH according to Table 1 (Steps 5-7) to give For-Tyr-Met-Gly-Trp-Met-Asp-MePhe-$OCH_2$-Pam-resin which was sulfated and cleaved from the resin according to Table 1 (Steps 21-25 and then Steps 26-29 with ammonia) to give the title compound which was chromatographically purified on Amberlite XAD-2, Trisacryl M DEAE, and P-40 ODS-3, sequentially, according to Table 1 (Step 30) to give 78 mg of the ammonium salt of the title compound. Amino acid analysis following acid decomposition gave Asp 1.02 (1), Tyr 1.01 (1), Met 1.95 (2), and Gly 1.02 (1). Infrared absorption spectrum showed a strong peak typical of a sulfuric acid ester at 1050 $cm^{-1}$. TLC $R_f$ 0.45.

EXAMPLE 25

iBuOCO-Tyr($SO_3H$)-Met-Gly-Trp-Met-Asp-MePhe-$NH_2$

H-MePhe-$OCH_2$-Pam-resin (Example 12) was sequentially coupled with Fmoc-Met-Asp(OtBu)-OH (Example 10), Fmoc-Trp-OH, Fmoc-Gly-OH, Fmoc-Met-OH, and Fmoc-Tyr(tBu)-OH according to Table 1 (coupling Steps 5-7 followed by Fmoc removal Steps 16-20) to give H-Tyr(tBu)-Met-Gly-Trp-Met-Asp(OtBu)-MePhe-$OCH_2$-Pam-resin which was coupled with iBuOCO-OSu (Example 1) according to Table 1 (Steps 8-9) to give iBuOCO-Tyr(tBu)-Met-Gly-Trp-Met-Asp(OtBu)-MePhe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2, Trisacryl M DEAE, and P-40 ODS-3, sequentially, according to Table 1 (Step 30) to give 302 mg of the ammonium salt of the title compound. Amino acid analysis following acid decomposition gave Asp 1.03 (1), Tyr 1.02 (1), Met 1.91 (2), and Gly 1.04 (1). Infrared absorption spectrum showed a strong peak typical of a sulfuric acid ester at 1050 $cm^{-1}$. TLC $R_f$ 0.67.

EXAMPLE 26

Hpp($SO_3H$)-Met-Gly-Trp-Met-Asp-MePhe-$NH_2$

H-MePhe-$OCH_2$-Pam-resin (Example 12) was sequentially coupled with Fmoc-Met-Asp(OtBu)-OH (Example 10), Fmoc-Trp-OH, Fmoc-Gly-OH, and Fmoc-Met-OH according to Table 1 (coupling Steps 3-4 followed by Fmoc removal Steps 16-20) to give H-Met-Gly-Trp-Met-Asp(OtBu)-MePhe-$OCH_2$-Pam-resin which was coupled with 3-(4-hydroxyphenyl)propionic acid N-hydroxysuccinimide ester (Hpp-OSu) according to Table 1 (Steps 8-9) to give Hpp-Met-Gly-Trp-Met-Asp(OtBu)-MePhe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Trisacryl M DEAE and P-40 ODS-3, sequentially, according to Table 1 (Step 30) to give 170 mg of the ammonium salt of the title compound. Amino acid analysis following acid decomposition gave Asp 1.02 (1), Met 1.97 (2), and Gly 1.01 (1). Infrared absorption spectrum showed a strong peak typical of a sulfuric acid ester at 1050 $cm^{-1}$. TLC $R_f$ 0.58.

EXAMPLE 27

PrOCO-Tyr($SO_3$H)-Met-Gly-Trp-Met-Asp-Phe-$NH_2$

H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, and Fmoc-Tyr(tBu)-OH according to Table 1 (coupling Steps 5-7 followed by Fmoc removal Steps 16-20) to give H-Tyr-(tBu)-Met-Gly-Trp-Met-Asp(OtBu)-Phe-$OCH_2$-Pam-resin which was coupled with PrOCO-OSu (mp 31-34 °C, prepared according to the procedure of Example 1 except that propyl chloroformate was substituted for isobutyl chloroformate) according to Table 1 (Steps 8-9) to give PrOCO-Tyr(tBu)-Met-Gly-Trp-Met-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 270 mg of the ammonium salt of the title compound. Amino acid analysis following acid decomposition gave Asp 1.01 (1), Tyr 1.03 (1), Met 1.87 (2), Gly 1.02 (1), and Phe 1.06 (1). Infrared absorption spectrum showed a strong peak typical of a sulfuric acid ester at 1050 $cm^{-1}$. TLC $R_f$ 0.45.

EXAMPLE 28

EtOCO-Tyr($SO_3$H)-Met-Gly-Trp-Met-Asp-Phe-$NH_2$

H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, and Fmoc-Tyr(tBu)-OH according to Table 1 (coupling Steps 5-7 followed by Fmoc removal Steps 16-20) to give H-Tyr-(tBu)-Met-Gly-Trp-Met-Asp(OtBu)-Phe-$OCH_2$-Pam-resin which was coupled with EtOCO-OSu (mp 52-54.5 °C, prepared according to the procedure of Example 1 except that ethyl chloroformate was substituted for isobutyl chloroformate) according to Table 1 (Steps 8-9) to give EtOCO-Tyr(tBu)-Met-Gly-Trp-Met-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 300 mg of the ammonium salt of the title compound. Amino acid analysis following acid decomposition gave Asp 1.02 (1), Tyr 1.01 (1), Met 1.91 (2), Gly 1.02 (1), and Phe 1.04 (1). Infrared absorption spectrum showed a strong peak typical of a sulfuric acid ester at 1050 $cm^{-1}$. TLC $R_f$ 0.44.

EXAMPLE 29

MeOCO-Tyr($SO_3$H)-Met-Gly-Trp-Met-Asp-Phe-$NH_2$

H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, and Fmoc-Tyr(tBu)-OH according to Table 1 (coupling Steps 5-7 followed by Fmoc removal Steps 16-20) to give H-Tyr-(tBu)-Met-Gly-Trp-Met-Asp(OtBu)-Phe-$OCH_2$-Pam-resin which was coupled with MeOCO-OSu (mp 87-89 °C, prepared according to the procedure of Example 1 except that methyl chloroformate was substituted for isobutyl chloroformate) according to Table 1 (Steps 8-9) to give MeOCO-Tyr(tBu)-Met-Gly-Trp-Met-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 270 mg of the ammonium salt of the title compound. Amino acid analysis following acid decomposition gave Asp 1.04 (1), Tyr 1.05 (1), Met 1.82 (2), Gly 1.03 (1), and Phe 1.06 (1). Infrared absorption spectrum showed a strong peak typical of a sulfuric acid ester at 1050 $cm^{-1}$. TLC $R_f$ 0.44.

EXAMPLE 30

H-$\beta$Asp-Tyr($SO_3$H)-Met-Gly-Trp-Met-Asp-Phe-$NH_2$

The title compound has previously been prepared (Digestive Diseases, 15, 149-156 (1970)). H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, Fmoc-Tyr-OH (Example 3), and Boc-$\beta$Asp(OtBu)-OH according to Table 1

(coupling Steps 3-4 followed by Fmoc removal Steps 16-20) to provide Boc-βAsp(OtBu)-Tyr-Met-Gly-Trp-Met-Asp(OtBu)-Phe-$OCH_2$-Pam-resin which was sulfated, deprotected, and cleaved from the resin according to Table 1 (Steps 21-25, Steps 10-15, and then Steps 26-29 with ammonia) to give the title compound which was chromatographically purified on Trisacryl M DEAE according to Table 1 (Step 30) to give 283 mg of the ammonium salt of the title compound. Amino acid analysis following acid decomposition gave Asp 2.03 (2), Tyr 0.94 (1), Met 2.08 (2), Gly 0.99 (1), and Phe 0.96 (1). Infrared absorption spectrum showed a strong peak typical of a sulfuric acid ester at 1050 $cm^{-1}$. TLC $R_f$ 0.52.

EXAMPLE 31

Suc-Tyr($SO_3H$)-Met-Gly-Trp-Met-Asp-Phe-$NH_2$

The title compound has previously been prepared (European Patent Application 0107860). H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, Fmoc-Met-OH, and Fmoc-Tyr-OH (Example 3) according to Table 1 (coupling Steps 3-4 followed by Fmoc removal Steps 16-20) to provide H-Tyr-Met-Gly-Trp-Met-Asp(OtBu)-Phe-$OCH_2$-Pam-resin which was coupled with succinic anhydride in DMF according to Table 1 (Steps 8-9) to give Suc-Try-Met-Gly-Trp-Met-Asp(OtBu)-Phe-$OCH_2$-Pam-resin which was sulfated, deprotected, and cleaved from the resin according to Table 1 (Steps 21-25, Steps 10-15, and then Steps 26-29 with ammonia) to give the title compound which was chromatographically purified on Trisacryl M DEAE according to Table 1 (Step 30) to give 246 mg of the ammonium salt of the title compound. Amino acid analysis following acid decomposition gave Asp 1.03 (1), Tyr 0.95 (1), Met 2.08 (2), Gly 0.98 (1), and Phe 0.96 (1). Infrared absorption spectrum showed a strong peak typical of a sulfuric acid ester at 1050 $cm^{-1}$. TLC $R_f$ 0.54.

EXAMPLE 32

Hpp($SO_3H$)-Met-Gly-Trp-Met-Asp-Phe-$NH_2$

The title compound has previously been prepared (Int. J. Peptide Protein Res., 16, 402-411 (1980)). H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, and Fmoc-Met-OH according to Table 1 (coupling Steps 3-4 followed by Fmoc removal Steps 16-20) to provide H-Met-Gly-Trp-Met-Asp (OtBu)-Phe-$OCH_2$-Pam-resin which was coupled with Hpp-OSu in DMF according to Table 1 (Step 8-9) to give Hpp-Met-Gly-Trp-Met-Asp (OtBu)-Phe-$OCH_2$-Pam-resin which was sulfated, deprotected, and cleaved from the resin according to Table 1 (Steps 21-25, Steps 10-15, and then Steps 26-29 with ammonia) to give the title compound which was chromatographically purified on Trisacryl M DEAE according to Table 1 (Step 30) to give 65 mg of the ammonium salt of the title compound. Amino acid analysis following acid decomposition gave Asp 1.04 (1), Met 2.04 (2), Gly 0.95 (1), and Phe 1.00 (1). Infrared absorption spectrum showed a strong peak typical of a sulfuric acid ester at 1050 $cm^{-1}$. TLC $R_f$ 0.47.

EXAMPLE 33

For-Tyr($SO_3H$)-Met-Gly-Trp-Met-Asp-Phe-$NH_2$

The title compound has previously been prepared (U.S. Patent 3,705,140). H-Phe-$OCH_2$-Pam-resin (Example 11) was sequentially coupled with Fmoc-Asp(OtBu)-OH, Fmoc-Met-OH, Fmoc-Trp-OH, Fmoc-Gly-OH, and Fmoc-Met-OH according to Table 1 (coupling Steps 3-4 followed by Fmoc removal Steps 16-20) to provide H-Met-Gly-Trp-Met-Asp(OtBu)-Phe-$OCH_2$-Pam-resin which was deprotected according to Table 1 (Steps 10-15) to provide H-Met-Gly-Trp-Met-Asp-Phe-$OCH_2$-Pam-resin which was coupled with For-Tyr-OH according to Table 1 (Step 3-4) to give For-Tyr-Met-Gly-Trp-Met-Asp-Phe-$OCH_2$-Pam-resin which was sulfated and cleaved from the resin according to Table 1 (Steps 21-25 and then Steps 26-29 with ammonia) to give the title compound which was chromatographically purified on Trisacryl M DEAE and P-40 ODS-3, sequentially, according to Table 1 (Step 30) to give 30 mg of the ammonium salt of the title compound. Amino acid analysis following acid decomposition gave Asp 1.00 (1), Met 1.98 (2), Gly 1.07 (1), and Phe 1.00 (1). Infrared absorption spectrum showed a strong peak typical of a sulfuric acid ester at 1050 $cm^{-1}$. TLC $R_f$ 0.37.

EXAMPLE 34

Suc-Tyr($SO_3H$)-Ahx-Gly-Trp-Ahx-Asp-MePhe-$NH_2$

H-MePhe-$OCH_2$-Pam-resin (Example 12) was sequentially coupled with Fmoc-Ahx-Asp(OtBu)-OH (Example 10, Fmoc-Met-OH replaced with Fmoc-Ahx-OH), Fmoc-Trp-OH, Fmoc-Gly-OH, Fmoc-Ahx-OH, and Fmoc-Tyr(tBu)-OH according to Table 1 (coupling Steps 5-7 followed by Fmoc removal Steps 16-20) to provide H-Tyr(tBu)-Ahx-Gly-Trp-Ahx-Asp(OtBu)-MePhe-$OCH_2$-Pam-resin which was coupled with succinic anhydride in DMF according to Table 1 (Steps 8-9) to give Suc-Tyr(tBu)-Ahx-Gly-Trp-Ahx-Asp(OtBu)-MePhe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2, Trisacryl M DEAE, and P-40 ODS-3, sequentially, according to Table 1 (Step 30) to give 100 mg of the ammonium salt of the title compound. Amino acid analysis following acid decomposition gave Tyr 0.97 (1), Gly 1.00 (1), and Asp 1.00 (1). Infrared absorption spectrum showed a strong peak typical of a sulfuric acid ester at 1050 $cm^{-1}$. TLC $R_f$ 0.46.

EXAMPLE 35

iBuOCO-Tyr($SO_3H$)-Ahx-Gly-Trp-Ahx-Asp-MePhe-$NH_2$

H-MePhe-$OCH_2$-Pam-resin (Example 12) was sequentially coupled with Fmoc-Ahx-Asp(OtBu)-OH (Example 10, Fmoc-Met-OH replaced with Fmoc-Ahx-OH), Fmoc-Trp-OH, Fmoc-Gly-OH, Fmoc-Ahx-OH, and Fmoc-Tyr(tBu)-OH according to Table 1 (coupling Steps 5-7 followed by Fmoc removal Steps 16-20) to provide H-Tyr(tBu)-Ahx-Gly-Trp-Ahx-Asp(OtBu)-MePhe-$OCH_2$-Pam-resin which was coupled with iBuOCO-OSu (EXAMPLE 1) in DMF according to Table 1 (Steps 8-9) to give iBuOCO-Tyr(tBu)-Ahx-Gly-Trp-Ahx-Asp(OtBu)-MePhe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 290 mg of the ammonium salt of the title compound. Infrared absorption spectrum showed a strong peak typical of a sulfuric acid ester at 1050 $cm^{-1}$. TLC $R_f$ 0.54.

EXAMPLE 36

Hpp($SO_3H$)-Ahx-Gly-Trp-Ahx-Asp-MePhe-$NH_2$

H-MePhe-$OCH_2$-Pam-resin (Example 12) was sequentially coupled with Fmoc-Ahx-Asp(OtBu)-OH (Example 10, Fmoc-Met-OH replaced with Fmoc-Ahx-OH), Fmoc-Trp-OH, Fmoc-Gly-OH, and Fmoc-Ahx-OH according to Table 1 (coupling Steps 5-7 followed by Fmoc removal Steps 16-20) to provide H-Ahx-Gly-Trp-Ahx-Asp-(OtBu)-MePhe-$OCH_2$-Pam-resin which was coupled with Hpp-OSu in DMF according to Table 1 (Steps 8-9) to give Hpp-Ahx-Gly-Trp-Ahx-Asp(OtBu)-MePhe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2, Trisacryl M DEAE, and P-40 ODS-3, sequentially, according to Table 1 (Step 30) to give 91 mg of the ammonium salt of the title compound. Amino acid analysis following acid decomposition gave Ahx 1.92 (2), Gly 1.03 (1), and Asp 1.05 (1). Infrared absorption spectrum showed a strong peak typical of a sulfuric acid ester at 1050 $cm^{-1}$. TLC $R_f$ 0.40.

EXAMPLE 37

iBuOCO-Tyr($SO_3H$)-Ahx-Gly-Trp-Ahx-Asp-Phe-$NH_2$

H-Phe-$OCH_2$-Pam-resin (Example 11) was sequentially coupled with Fmoc-Asp(OtBu)-OH, Fmoc-Ahx-OH, Fmoc-Trp-OH, Fmoc-Gly-OH, Fmoc-Ahx-OH, and Fmoc-Tyr(tBu)-OH according to Table 1 (coupling Steps 5-7 followed by Fmoc removal Steps 16-20) to provide H-Tyr(tBu)-Ahx-Gly-Trp-Ahx-Asp(OtBu)-Phe-$OCH_2$-Pam-resin which was coupled with iBuOCO-OSu (Example 1) in DMF according to Table 1 (Steps 8-9) to give iBuOCO-Tyr(tBu)-Ahx-Gly-Trp-Ahx-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40

ODS-3, sequentially, according to Table 1 (Step 30) to give 800 mg of the ammonium salt of the title compound. Amino acid analysis following acid decomposition gave Tyr 0.90 (1), Ahx 1.84 (2), Gly 1.12(1), Asp 1.12 (1), and Phe 0.92 (1). Infrared absorption spectrum showed a strong peak typical of a sulfuric acid ester at 1050 $cm^{-1}$. TLC $R_f$ 0.58.

EXAMPLE 38

Hpp($SO_3H$)-Ahx-Gly-Trp-Ahx-Asp-Phe-$NH_2$

H-Phe-$OCH_2$-Pam-resin (Example 11) was sequentially coupled with Fmoc-Asp(OtBu)-OH, Fmoc-Ahx-OH, Fmoc-Trp-OH, Fmoc-Gly-OH, and Fmoc-Ahx-OH according to Table 1 (coupling Steps 5-7 followed by Fmoc removal Steps 16-20) to provide H-Ahx-Gly-Trp-Ahx-Asp-(OtBu)-Phe-$OCH_2$-Pam-resin which was coupled with Hpp-OSu in DMF according to Table 1 (Step 8-9) to give Hpp-Ahx-Gly-Trp-Ahx-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 450 mg of the ammonium salt of the title compound. Amino acid analysis following acid decomposition gave Ahx 1.92 (2), Gly 1.01(1), Asp 1.10(1), and Phe 0.97(1). Infrared absorption spectrum showed a strong peak typical of a sulfuric acid ester at 1050 $cm^{-1}$. TLC $R_f$ 0.47.

EXAMPLE 39

For-Tyr($SO_3H$)-Ile-Gly-Trp-Ile-Asp-Phe-$NH_2$

H-Phe-$OCH_2$-Pam-resin (Example 11) was sequentially coupled with Fmoc-Ile-Asp(OtBu)-OH (Example 10, Fmoc-Met-OH replaced with Fmoc-Ile-OH), Fmoc-Trp-OH, Fmoc-Gly-OH, and Fmoc-Ile-OH according to Table 1 (coupling Steps 5-7 followed by Fmoc removal Steps 16-20) to provide H-Ile-Gly-Trp-Ile-Asp(OtBu)-Phe-$OCH_2$-Pam-resin which was deprotected according to Table 1 (Steps 10-15) to provide H-Ile-Gly-Trp-Ile-Asp-Phe-$OCH_2$-Pam-resin which was coupled with For-Tyr-OH according to Table 1 (Steps 3-4) to give For-Tyr-Ile-Gly-Trp-Ile-Asp-Phe-$OCH_2$-Pam-resin which was sulfated and cleaved from the resin according to Table 1 (Steps 21-25 and then Steps 26-29 with ammonia) to give the title compound which was chromatographically purified on Amberlite XAD-2 and P-40 ODS-3, sequentially, according to Table 1 (Step 30) to give 200 mg of the ammonium salt of the title compound. Amino acid analysis following acid decomposition gave Tyr 0.98 (1), Ile 1.94 (2), Gly 1.00 (1), Asp 1.09 (1), and Phe 0.98 (1). Infrared absorption spectrum showed a strong peak typical of a sulfuric acid ester at 1050 $cm^{-1}$. TLC $R_f$ 0.59.

EXAMPLE 40

Suc-Tyr($SO_3H$)-Ile-Gly-Trp-Ile-Asp-Phe-$NH_2$

H-Phe-$OCH_2$-Pam-resin (Example 11) was sequentially coupled with Fmoc-Ile-Asp(OtBu)-OH (Example 10, Fmoc-Met-OH replaced with Fmoc-Ile-OH), Fmoc-Trp-OH, Fmoc-Gly-OH, Fmoc-Ile-OH, and Fmoc-Tyr(tBu)-OH according to Table 1 (coupling Steps 5-7 followed by Fmoc removal Steps 16-20) to provide H-Tyr(tBu)-Ile-Gly-Trp-Ile-Asp(OtBu)-Phe-$OCH_2$-Pam-resin which was coupled with succinic anhydride in DMF according to Table 1 (Steps 8-9) to give Suc-Tyr(tBu)-Ile-Gly-Trp-Ile-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and P-40 ODS-3, sequentially, according to Table 1 (Step 30) to give 300 mg of the ammonium salt of the title compound. Amino acid analysis following acid decomposition gave Tyr 1.00 (1), Ile 2.07 (2), Gly 0.97 (1), Asp 0.97 (1), and Phe 0.99 (1). Infrared absorption spectrum showed a strong peak typical of a sulfuric acid ester at 1050 $cm^{-1}$. TLC $R_f$ 0.42.

EXAMPLE 41

iBuOCO-Tyr($SO_3H$)-Ile-Gly-Trp-Ile-Asp-Phe-$NH_2$

H-Phe-$OCH_2$-Pam-resin (Example 11) was sequentially coupled with Fmoc-Ile-Asp(OtBu)-OH (Example 10, Fmoc-Met-OH replaced with Fmoc-Ile-OH), Fmoc-Trp-OH, Fmoc-Gly-OH, Fmoc-Ile-OH, and Fmoc-Tyr(tBu)-OH according to Table 1 (coupling Steps 5-7 followed by Fmoc removal Steps 16-20) to provide H-Tyr(tBu)-Ile-Gly-Trp-Ile-Asp(OtBu)-Phe-$OCH_2$-Pam-resin which was coupled with iBuOCO-OSu (Example 1) in DMF according to Table 1 (Steps 8-9) to give iBuOCO-Tyr(tBu)-Ile-Gly-Trp-Ile-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amberlite XAD-2 and p-40 ODS-3, sequentially, according to Table 1 (Step 30) to give 390 mg of the ammonium salt of the title compound. Amino acid analysis following acid decomposition gave Tyr 1.07 (1), Ile 2.21 (2), Gly 1.04 (1), Asp 1.00 (1), and Phe 1.03 (1). Infrared absorption spectrum showed a strong peak typical of a sulfuric acid ester at 1050 $cm^{-1}$. TLC $R_f$ 0.61.

EXAMPLE 42

Hpp($SO_3H$)-Ile-Gly-Trp-Ile-Asp-Phe-$NH_2$

H-Phe-$OCH_2$-Pam-resin (Example 11) was sequentially coupled with Fmoc-Ile-Asp(OtBu)-OH (Example 10, Fmoc-Met-OH replaced with Fmoc-Ile-OH), Fmoc-Trp-OH, Fmoc-Gly-OH, and Fmoc-Ile-OH according to Table 1 (coupling Steps 5-7 followed by Fmoc removal Steps 16-20) to provide H-Ile-Gly-Trp-Ile-Asp(OtBu)-Phe-$OCH_2$-Pam-resin which was coupled with Hpp-OSu in DMF according to Table 1 (Step 8-9) to give Hpp-Ile-Gly-Trp-Ile-Asp(OtBu)-Phe-$OCH_2$-Pam-resin which was deprotected, sulfated, and cleaved from the resin according to Table 1 (Steps 10-15, Steps 21-25, and then Steps 26-29 with ammonia) to give the title compound which was chromatographically purified on Amber lite XAD-2 and P-40 ODS-3, sequentially, according to Table 1 (Step 30) to give 280 mg of the ammonium salt of the title compound. Amino acid analysis following acid decomposition gave Ile 2.04 (2), Gly 0.99 (1), Asp 0.98 (1), and Phe 0.99 (1). Infrared absorption spectrum showed a strong peak typica of a sulfuric acid ester at 1050 $cm^{-1}$. TLC $R_f$ 0.52.

## Claims

**Claims for the following Contracting States : BE, CH, LI, DE, FR, GB, IT, LU, NL, SE**

**1.** A peptide of the formula

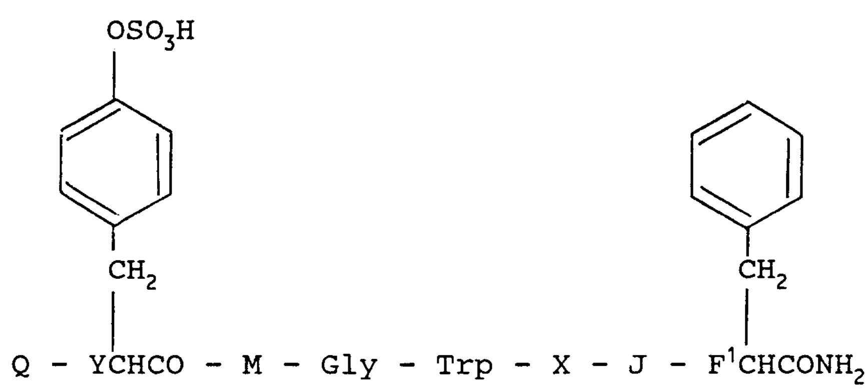

wherein
Q is H-$\beta$Asp, H-DAsp, For, Ac, Suc, desQ or $R^1R^2$CHOCO;
Y is H, (S)-NH or (S)-$R^3$N;
M is Met, Ahx, Leu or Ile;
X is Met, Ahx, Leu or Ile;
J is Asp, DAsp or MeAsp;
$F^1$ is (S)-NH or (S)-$R^4$N;
$R^1$ and $R^2$ independently represent H or lower alkyl;
$R^3$ and $R^4$ represent lower alkyl;
and pharmaceutically acceptable salts thereof;
provided that:
(1) Q is desQ when Y is H;
(2) Q is not Ac if, in the same peptide: Y is (S)-NH; M is Met, Ahx or Leu; X is Met, Ahx or Leu; J is Asp; and $F^1$ is (S)-NH;

(3) Q is neither H-$\beta$Asp nor For if, in the same peptide: Y is (S)-NH; M is Met, Ahx or Leu; X is Met, Ahx or Leu; J is Asp; and $F^1$ is (S)-NH;
(4) Y is not H if, in the same peptide: M is Met; X is Met; J is Asp; and $F^1$ is (S)-NH;
(5) Q is not Suc if, in the same peptide: Y is (S)-NH; M is Met; X is Met; and J is Asp; and
(6) Q is not H-DAsp if, in the same peptide: Y is (S)-NH; M is Met; X is Met; J is Asp; and $F^1$ is (S)-NH.

**2.** A peptide as claimed in claim 1, which is
iBuOCO-Tyr($SO_3H$)-Met-Gly-Trp-Met-Asp-Phe-$NH_2$,
or a pharmaceutically acceptable salt thereof.

**3.** A peptide as claimed in claim 1, which is
Suc-Tyr($SO_3H$)-Ahx-Gly-Trp-Ahx-Asp-Phe-$NH_2$,
or a pharmaceutically acceptable salt thereof.

**4.** A peptide as claimed in claim 1, which is
H-$\beta$Asp-Tyr($SO_3H$)-Met-Gly-Trp-Met-Asp-MePhe-$NH_2$,
or a pharmaceutically acceptable salt thereof.

**5.** A peptide as claimed in claim 1, which is
H-DAsp-Tyr($SO_3H$)-Met-Gly-Trp-Met-Asp-MePhe-$NH_2$,
or a pharmaceutically acceptable salt thereof.

**6.** A peptide as claimed in claim 1, which is
For-Tyr($SO_3H$)-Met-Gly-Trp-Met-Asp-MePhe-$NH_2$,
or a pharmaceutically acceptable salt thereof.

**7.** A peptide as claimed in claim 1, which is
iBuOCO-Tyr($SO_3H$)-Met-Gly-Trp-Met-Asp-MePhe-$NH_2$,
or a pharmaceutically acceptable salt thereof.

**8.** A peptide as claimed in claim 1, which is
Hpp($SO_3H$)-Met-Gly-Trp-Met-Asp-MePhe-$NH_2$,
or a pharmaceutically acceptable salt thereof.

**9.** A peptide as claimed in claim 1, which is
PrOCO-Tyr($SO_3H$)-Met-Gly-Trp-Met-Asp-Phe-$NH_2$,
or a pharmaceutically acceptable salt thereof.

**10.** A peptide as claimed in claim 1, which is
EtOCO-Tyr($SO_3H$)-Met-Gly-Trp-Met-Asp-Phe-$NH_2$,
or a pharmaceutically acceptable salt thereof.

**11.** A peptide as claimed in claim 1, which is
MeOCO-Tyr($SO_3H$)-Met-Gly-Trp-Met-Asp-Phe-$NH_2$,
or a pharmaceutically acceptable salt thereof.

**12.** A peptide as claimed in claim 1, which is
Suc-Tyr($SO_3H$)-Ahx-Gly-Trp-Ahx-Asp-MePhe-$NH_2$,
or a pharmaceutically acceptable salt thereof.

**13.** A peptide as claimed in claim 1, which is
iBuOCO-Tyr($SO_3H$)-Ahx-Gly-Trp-Ahx-Asp-MePhe-$NH_2$,
or a pharmaceutically acceptable salt thereof.

**14.** A peptide as claimed in claim 1, which is
Hpp($SO_3H$)-Ahx-Gly-Trp-Ahx-Asp-MePhe-$NH_2$,
or a pharmaceutically acceptable salt thereof.

**15.** A peptide as claimed in claim 1, which is
$iBuOCO-Tyr(SO_3H)-Ahx-Gly-Trp-Ahx-Asp-Phe-NH_2$,
or a pharmaceutically acceptable salt thereof.

**16.** A peptide as claimed in claim 1, which is
$Hpp(SO_3H)-Ahx-Gly-Trp-Ahx-Asp-Phe-NH_2$,
or a pharmaceutically acceptable salt thereof.

**17.** A peptide as claimed in claim 1, which is
$For-Tyr(SO_3H)-Ile-Gly-Trp-Ile-Asp-Phe-NH_2$,
or a pharmaceutically acceptable salt thereof.

**18.** A peptide as claimed in claim 1, which is
$Suc-Tyr(SO_3H)-Ile-Gly-Trp-Ile-Asp-Phe-NH_2$,
or a pharmaceutically acceptable salt thereof.

**19.** A peptide as claimed in claim 1, which is
$iBuOCO-Tyr(SO_3H)-Ile-Gly-Trp-Ile-Asp-Phe-NH_2$,
or a pharmaceutically acceptable salt thereof.

**20.** A peptide as claimed in claim 1, which is
$Hpp(SO_3H)-Ile-Gly-Trp-Ile-Asp-Phe-NH_2$,
or a pharmaceutically acceptable salt thereof.

**21.** A composition for preventing obesity comprising a peptide as defined in claim 1, but without provisos (2)-(6).

**22.** The use of a peptide as defined in claim 1, as a pharmaceutical.

**23.** The use of a peptide as defined in claim 1, but without provisos (2)-(6), in the manufacture of a medicament for the treatment of obesity.

**24.** A method of improving the bodily appearance of a human being which comprises administering to that human being intraperitoneally, intravenously, intramuscularly, subcutaneously or intranasally a peptide as defined in claim 1, but without provisos (2)-(6), or a pharmaceutically acceptable salt thereof, until a cosmetically beneficial loss of body weight has occurred.

**25.** A process for the production of a peptide as defined in claim 1 by the solid phase method of sequential addition (followed by deprotection after each addition) of the requisite protected amino acid or protected oligopeptide to an amino-acyl resin of the formula

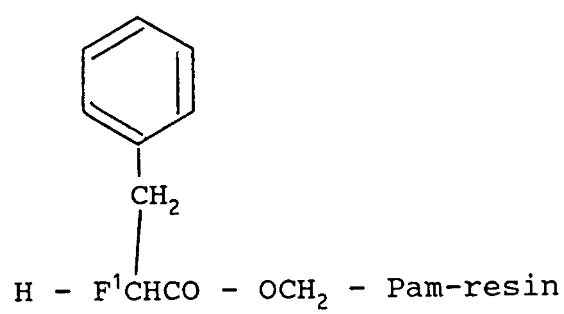

wherein $F^1$ is as defined in claim 1,
to afford a protected peptidyl resin of the formula

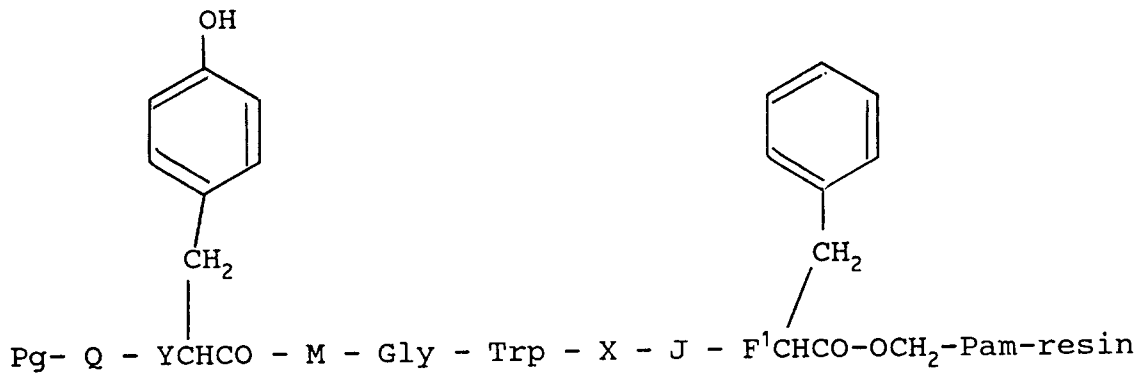

wherein Pg is a suitable protecting group and Q, Y, M, X, J and $F^1$ are as defined in claim 1, which is sulphated, deprotected and treated with a methanolic amine.

**26.** A process for the production of a peptide as defined in claim 1 by the solution phase method of sequential addition (followed by deprotection after each addition) of the requisite protected amino acid or protected oligopeptide to an amino-acyl amide of the formula

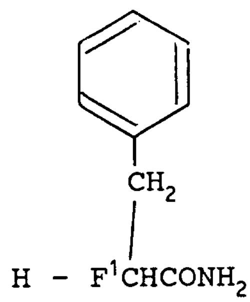

wherein $F^1$ is as defined in claim 1, to afford a protected peptide amide of the formula

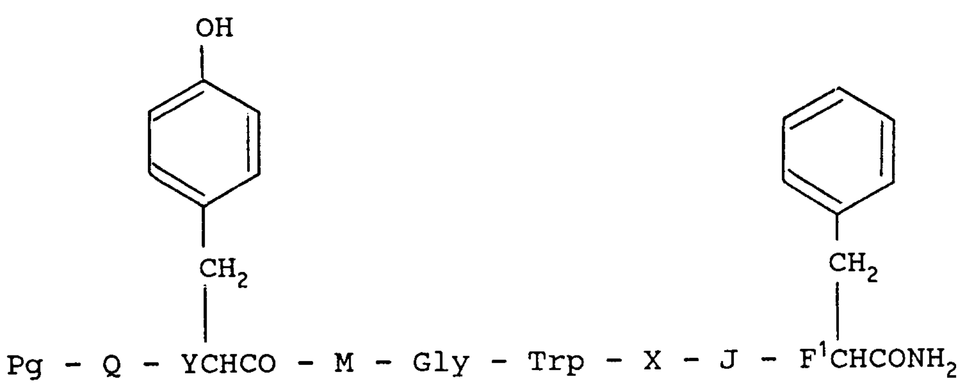

wherein Pg is a suitable protecting group and Q, Y, M, X, J and $F^1$ are as defined in claim 1, which is sulphated and deprotected.

**Claims for the following Contracting States : AT, ES, GR,**

**1.** A process for preparation of a peptide of the formula

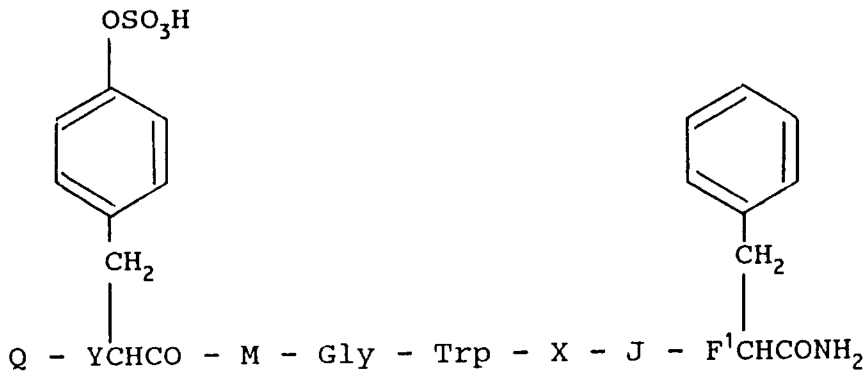

wherein
Q is H-βAsp, H-DAsp, For, Ac, Suc, desQ or $R^1R^2$CHOCO;
Y is H, (S)-NH or (S)-$R^3$N;
M is Met, Ahx, Leu or Ile;
X is Met, Ahx, Leu or Ile;
J is Asp, DAsp or MeAsp;
$F^1$ is (S)-NH or (S)-$R^4$N;
$R^1$ and $R^2$ independently represent H or lower alkyl;
$R^3$ and $R^4$ represent lower alkyl;
and pharmaceutically acceptable salts thereof;
provided that:
(1) Q is desQ when Y is H;
(2) Q is not Ac if, in the same peptide: Y is (S)-NH; M is Met, Ahx or Leu; X is Met, Ahx or Leu; J is Asp; and $F^1$ is (S)-NH;
(3) Q is neither H-βAsp nor For if, in the same peptide: Y is (S)-NH; M is Met, Ahx or Leu; X is Met, Ahx or Leu; J is Asp; and $F^1$ is (S)-NH;
(4) Y is not H if, in the same peptide: M is Met; X is Met; J is Asp; and $F^1$ is (S)-NH;
(5) Q is not Suc if, in the same peptide: Y is (S)-NH; M is Met; X is Met; and J is Asp; and
(6) Q is not H-DAsp if, in the same peptide: Y is (S)-NH; M is Met; X is Met; J is Asp; and $F^1$ is (S)-NH
by the solid phase method of sequential addition (followed by deprotection after each addition) of the requisite protected amino acid or protected oligopeptide to an amino-acyl resin of the formula

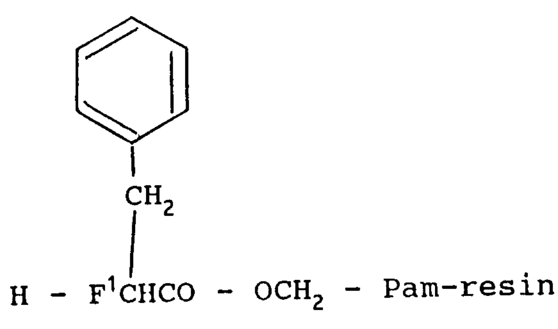

wherein $F^1$ is as defined above,
to afford a protected peptidyl resin of the formula

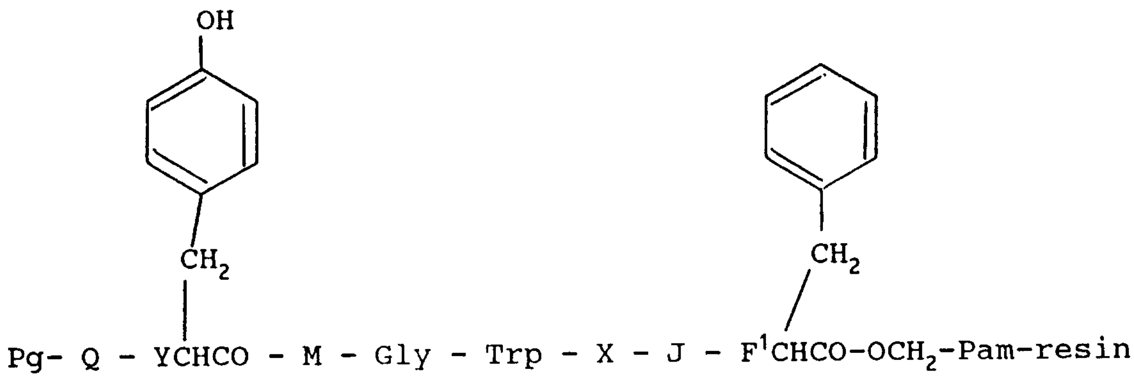

wherein Pg is a suitable protecting group and Q, Y, M, X, J and $F^1$ are as defined above, which is sulphated, deprotected and treated with a methanolic amine.

**2.** A process for the production of a peptide of formula

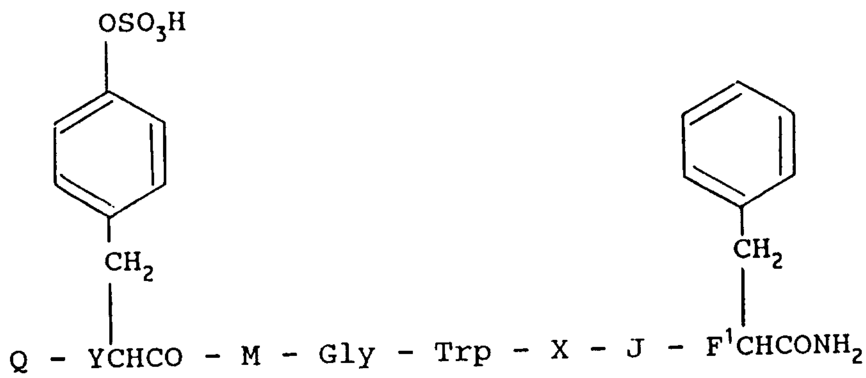

wherein
Q is H-βAsp, H-DAsp, For, Ac, Suc, desQ or $R^1R^2CHOCO$;
Y is H, (S)-NH or (S)-$R^3$N;
M is Met, Ahx, Leu or Ile;
X is Met, Ahx, Leu or Ile;
J is Asp, DAsp or MeAsp;
$F^1$ is (S)-NH or (S)-$R^4$N;
$R^1$ and $R^2$ independently represent H or lower alkyl;
$R^3$ and $R^4$ represent lower alkyl;
and pharmaceutically acceptable salts thereof;
provided that:
(1) Q is desQ when Y is H;
(2) Q is not Ac if, in the same peptide: Y is (S)-NH; M is Met, Ahx or Leu; X is Met, Ahx or Leu; J is Asp; and $F^1$ is (S)-NH;
(3) Q is neither H-βAsp nor For if, in the same peptide: Y is (S)-NH; M is Met, Ahx or Leu; X is Met, Ahx or Leu; J is Asp; and $F^1$ is (S)-NH;
(4) Y is not H if, in the same peptide: M is Met; X is Met; J is Asp; and $F^1$ is (S)-NH;
(5) Q is not Suc if, in the same peptide: Y is (S)-NH; M is Met; X is Met; and J is Asp; and
(6) Q is not H-DAsp if, in the same peptide: Y is (S)-NH; M is Met; X is Met; J is Asp; and $F^1$ is (S)-NH
by the solution phase method of sequential addition (followed by deprotection after each addition) of the requisite protected amino acid or protected oligopeptide to an amino-acyl amide of the formula

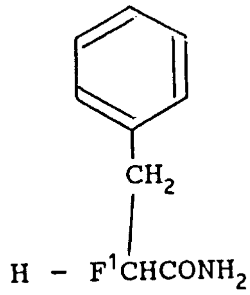

wherein $F^1$ is as defined above,
to afford a protected peptide amide of the formula

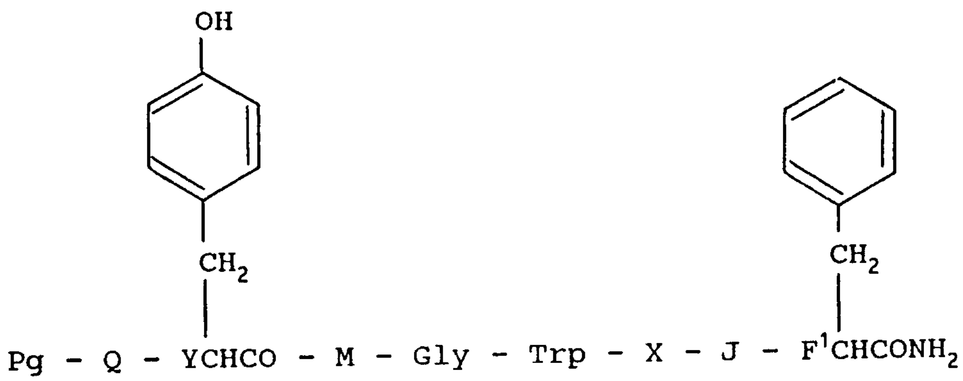

wherein Pg is a suitable protecting group and Q, Y, M, X, J and $F^1$ are as defined above, which is sulphated and deprotected.

3. A process, according to claim 1 or claim 2, wherein the peptide prepared is iBuOCO-Tyr($SO_3H$)-Met-Gly-Trp-Met-Asp-Phe-$NH_2$, or a pharmaceutically acceptable salt thereof.

4. A process, according to claim 1 or claim 2, wherein the peptide prepared is Suc-Tyr($SO_3H$)-Ahx-Gly-Trp-Ahx-Asp-Phe-$NH_2$, or a pharmaceutically acceptable salt thereof.

5. A process, according to claim 1 or claim 2, wherein the peptide prepared is H-$\beta$Asp-Tyr($SO_3H$)-Met-Gly-Trp-Met-Asp-MePhe-$NH_2$, or a pharmaceutically acceptable salt thereof.

6. A process, according to claim 1 or claim 2, wherein the peptide prepared is H-DAsp-Tyr($SO_3H$)-Met-Gly-Trp-Met-Asp-MePhe-$NH_2$, or a pharmaceutically acceptable salt thereof.

7. A process, according to claim 1 or claim 2, wherein the peptide prepared is For-Tyr($SO_3H$)-Met-Gly-Trp-Met-Asp-MePhe-$NH_2$, or a pharmaceutically acceptable salt thereof.

8. A process, according to claim 1 or claim 2, wherein the peptide prepared is iBuOCO-Tyr($SO_3H$)-Met-Gly-Trp-Met-Asp-MePhe-$NH_2$, or a pharmaceutically acceptable salt thereof.

9. A process, according to claim 1 or claim 2, wherein the peptide prepared is Hpp($SO_3H$)-Met-Gly-Trp-Met-Asp-MePhe-$NH_2$, or a pharmaceutically acceptable salt thereof.

**10.** A process, according to claim 1 or claim 2, wherein the peptide prepared is PrOCO-Tyr($SO_3H$)-Met-Gly-Trp-Met-Asp-Phe-$NH_2$, or a pharmaceutically acceptable salt thereof.

**11.** A process, according to claim 1 or claim 2, wherein the peptide prepared is EtOCO-Tyr($SO_3H$)-Met-Gly-Trp-Met-Asp-Phe-$NH_2$, or a pharmaceutically acceptable salt thereof.

**12.** A process, according to claim 1 or claim 2, wherein the peptide prepared is MeOCO-Tyr($SO_3H$)-Met-Gly-Trp-Met-Asp-Phe-$NH_2$, or a pharmaceutically acceptable salt thereof.

**13.** A process, according to claim 1 or claim 2, wherein the peptide prepared is Suc-Tyr($SO_3H$)-Ahx-Gly-Trp-Ahx-Asp-MePhe-$NH_2$, or a pharmaceutically acceptable salt thereof.

**14.** A process, according to claim 1 or claim 2, wherein the peptide prepared is iBuOCO-Tyr($SO_3H$)-Ahx-Gly-Trp-Ahx-Asp-MePhe-$NH_2$, or a pharmaceutically acceptable salt thereof.

**15.** A process, according to claim 1 or claim 2, wherein the peptide prepared is Hpp($SO_3H$)-Ahx-Gly-Trp-Ahx-Asp-MePhe-$NH_2$, or a pharmaceutically acceptable salt thereof.

**16.** A process, according to claim 1 or claim 2, wherein the peptide prepared is iBuOCO-Tyr($SO_3H$)-Ahx-Gly-Trp-Ahx-Asp-Phe-$NH_2$, or a pharmaceutically acceptable salt thereof.

**17.** A process, according to claim 1 or claim 2, wherein the peptide prepared is Hpp($SO_3H$)-Ahx-Gly-Trp-Ahx-Asp-Phe-$NH_2$, or a pharmaceutically acceptable salt thereof.

**18.** A process, according to claim 1 or claim 2, wherein the peptide prepared is For-Tyr($SO_3H$)-Ile-Gly-Trp-Ile-Asp-Phe-$NH_2$, or a pharmaceutically acceptable salt thereof.

**19.** A process, according to claim 1 or claim 2, wherein the peptide prepared is Suc-Tyr($SO_3H$)-Ile-Gly-Trp-Ile-Asp-Phe-$NH_2$, or a pharmaceutically acceptable salt thereof.

**20.** A process, according to claim 1 or claim 2, wherein the peptide prepared is iBuOCO-Tyr($SO_3H$)-Ile-Gly-Trp-Ile-Asp-Phe-$NH_2$, or a pharmaceutically acceptable salt thereof.

**21.** A process, according to claim 1 or claim 2, wherein the peptide prepared is Hpp($SO_3H$)-Ile-Gly-Trp-Ile-Asp-Phe-$NH_2$, or a pharmaceutically acceptable salt thereof.

**22.** A method of improving the bodily appearance of a human being which comprises administering to that human being intraperitoneally, intravenously, intramuscularly, subcutaneously or intranasally a peptide as defined in claim 1, but without provisos (2)-(6), or a pharmaceutically acceptable salt thereof, until a cosmetically beneficial loss of body weight has occurred.

**Patentansprüche**
**Patentansprüche für folgende Vertragsstaaten : BE, CH, LI, DE, FR, GB, IT, LU, NL, SE**

1. Peptid der Formel

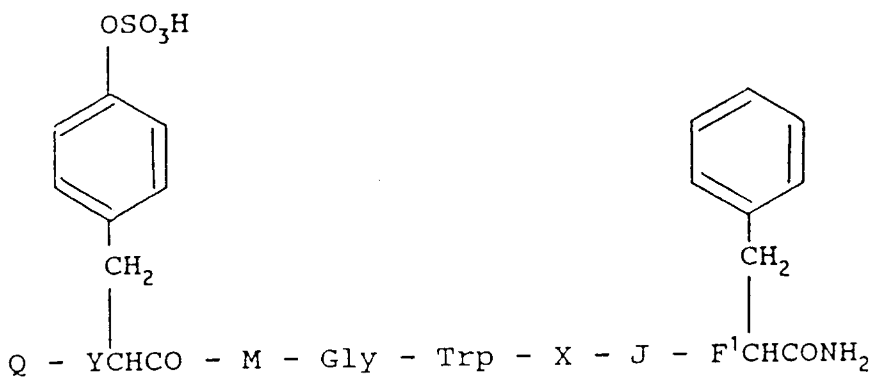

worin
Q H-$\beta$Asp, H-DAsp, For, Ac, Suc, desQ oder $R^1R^2CHOCO$ ist;
Y H, (S)-NH oder (S)-$R^3N$ ist;
M Met, Ahx, Leu oder Ile ist;
X Met, Ahx, Leu oder Ile ist;
J Asp, DAsp oder MeAsp ist;
$F^1$ (S)-NH oder (S)-$R^4$N ist;
$R^1$ und $R^2$ unabhängig voneinander H oder nied.Alkyl darstellen;
$R^3$ und $R^4$ nied.Alkyl darstellen;
und pharmazeutisch akzeptable Salze davon;
mit der Maßgabe, daß:
(1) Q desQ ist, wenn Y H ist;
(2) Q nicht Ac ist, wenn im selben Peptid: Y (S)-NH ist; M Met, Ahx oder Leu ist; X Met, Ahx oder Leu ist; J Asp ist; und $F^1$ (S)-NH ist;
(3) Q weder H-$\beta$Asp noch For ist, wenn im selben Peptid: Y (S)-NH ist; M Met, Ahx oder Leu ist; X Met, Ahx oder Leu ist; J Asp ist; und $F^1$ (S)-NH ist;
(4) Y nicht H ist, wenn im selben Peptid: M Met ist; X Met ist; J Asp ist; und $F^1$ (S)-NH ist;
(5) Q nicht Suc ist, wenn im selben Peptid: Y (S)-NH ist; M Met ist; X Met ist; und J Asp ist; und
(6) Q nicht H-DAsp ist, wenn im selben Peptid: Y (S)-NH ist; M Met ist; X Met ist; J Asp ist; und $F^1$ (S)-NH ist.

2. Peptid nach Anspruch 1, welches iBuOCO-Tyr($SO_3H$)-Met-Gly-Trp-Met-Asp-Phe-$NH_2$ oder ein pharmazeutisch akzeptables Salz davon ist.

3. Peptid nach Anspruch 1, welches Suc-Tyr($SO_3H$)-Ahx-Gly-Trp-Ahx-Asp-Phe-$NHC_2$ oder ein pharmazeutisch akzeptables Salz davon ist.

4. Peptid nach Anspruch 1, welches H-$\beta$Asp-Tyr($SO_3H$)-Met-Gly-Trp-Met-Asp-MePhe-$NH_2$ oder ein pharmazeutisch akzeptables Salz davon ist.

5. Peptid nach Anspruch 1, welches H-DAsp-Tyr($SO_3H$)-Met-Gly-Trp-Met-Asp-MePhe-$NH_2$ oder ein pharmazeutisch akzeptables Salz davon ist.

6. Peptid nach Anspruch 1, welches For-Tyr($SO_3H$)-Met-Gly-Trp-Met-Asp-MePhe-$NH_2$ oder ein pharmazeutisch akzeptables Salz davon ist.

7. Peptid nach Anspruch 1, welches iBuOCO-Tyr($SO_3H$)-Met-Gly-Trp-Met-Asp-MePhe-$NH_2$ oder ein pharmazeutisch akzeptables Salz davon ist.

8. Peptid nach Anspruch 1, welches Hpp($SO_3H$)-Met-Gly-Trp-Met-Asp-MePhe-$NH_2$ oder ein pharmazeutisch akzeptables Salz davon ist.

9. Peptid nach Anspruch 1, welches PrOCO-Tyr($SO_3H$)-Met-Gly-Trp-Met-Asp-Phe-$NH_2$ oder ein pharmazeutisch akzeptables Salz davon ist.

10. Peptid nach Anspruch 1, welches EtOCO-Tyr($SO_3H$)-Met-Gly-Trp-Met-Asp-Phe-$NH_2$ oder ein pharmazeutisch akzeptables Salz davon ist.

11. Peptid nach Anspruch 1, welches MeOCO-Tyr($SO_3H$)-Met-Gly-Trp-Met-Asp-Phe-$NH_2$ oder ein pharmazeutisch akzeptables Salz davon ist.

12. Peptid nach Anspruch 1, welches Suc-Tyr($SO_3H$)-Ahx-Gly-Trp-Ahx-Asp-MePhe-$NH_2$ oder ein pharmazeutisch akzeptables Salz davon ist.

13. Peptid nach Anspruch 1, welches iBuOCO-Tyr($SO_3H$)-Ahx-Gly-Trp-Ahx-Asp-MePhe-$NH_2$ oder ein pharmazeutisch akzeptables Salz davon ist.

14. Peptid nach Anspruch 1, welches Hpp($SO_3H$)-Ahx-Gly-Trp-Ahx-Asp-MePhe-$NH_2$ oder ein pharmazeutisch akzeptables Salz davon ist.

15. Peptid nach Anspruch 1, welches iBuOCO-Tyr($SO_3H$)-Ahx-Gly-Trp-Ahx-Asp-Phe-$NH_2$ oder ein pharmazeutisch akzeptables Salz davon ist.

16. Peptid nach Anspruch 1, welches Hpp($SO_3H$)-Ahx-Gly-Trp-Ahx-Asp-Phe-$NH_2$ oder ein pharmazeutisch akzeptables Salz davon ist.

17. Peptid nach Anspruch 1, welches For-Tyr($SO_3H$)-Ile-Gly-Trp-Ile-Asp-Phe-$NH_2$ oder ein pharmazeutisch akzeptables Salz davon ist.

18. Peptid nach Anspruch 1, welches Suc-Tyr($SO_3H$)-Ile-Gly-Trp-Ile-Asp-Phe-$NH_2$ oder ein pharmazeutisch akzeptables Salz davon ist.

19. Peptid nach Anspruch 1, welches iBuOCO-Tyr($SO_3H$)-Ile-Gly-Trp-Ile-Asp-Phe-$NH_2$ oder ein pharmazeutisch akzeptables Salz davon ist.

20. Peptid nach Anspruch 1, welches Hpp($SO_3H$)-Ile-Gly-Trp-Ile-Asp-Phe-$NH_2$ oder ein pharmazeutisch akzeptables Salz davon ist.

21. Zusammensetzung zur Verhinderung von Fettsucht, welche ein Peptid nach Anspruch 1, aber ohne den Maßgaben (2) - (6) umfaßt.

22. Verwendung eines Peptids nach Anspruch 1 als Pharmazeutikum.

23. Verwendung eines Peptids nach Anspruch 1, aber ohne den Maßgaben (2) - (6), bei der Herstellung eines Medikaments zur Behandlung von Fettsucht.

24. Verfahren zur Verbesserung der körperlichen Erscheinung eines Menschen, welches die intraperitoneale, intravenöse, intramuskuläre, subkutane oder intranasale Verabreichung eines Peptids nach Anspruch 1, aber ohne den Maßgaben (2) - (6), oder eines pharmazeutisch akzeptablen Salzes davon an diesen Menschen bis zum Eintreten eines kosmetisch günstigen Verlusts an Körpergewicht umfaßt.

25. Verfahren zur Herstellung eines Peptids nach Anspruch 1 mittels der Feststoffphasenmethode durch sequentielle Zugabe (jeweils mit Entschützung im Anschluß an jede Zugabe) der erforderlichen geschützten Aminosäure oder des erforderlichen geschützten Oligopeptids zu einem Aminoacylharz der Formel

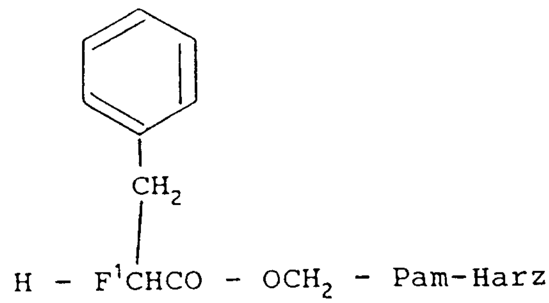

worin $F^1$ wie in Anspruch 1 definiert ist, zur Lieferung eines geschützten Peptidylharzes der Formel

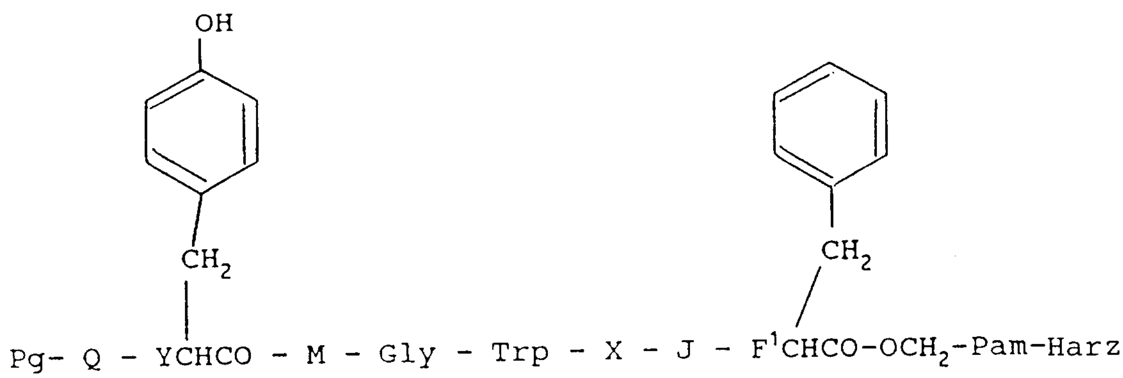

worin Pg eine geeignete Schutzgruppe ist und Q, Y, M, X, J und $F^1$ wie in Anspruch 1 definiert sind, welches sulfatiert, entschützt und mit einem Methanolamin behandelt wird.

**26.** Verfahren zur Herstellung eines Peptids nach Anspruch 1 mittels der Lösungsphasenmethode durch sequentielle Zugabe (jeweils mit Entschützung im Anschluß an jede Zugabe) der erforderlichen geschützten Aminosäure oder des erforderlichen geschützten Oligopeptids zu einem Aminoacylamid der Formel

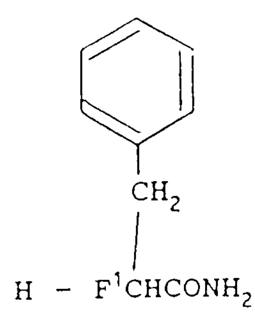

worin $F^1$ wie in Anspruch 1 definiert ist, zur Lieferung eines geschützten Peptidylamids der Formel

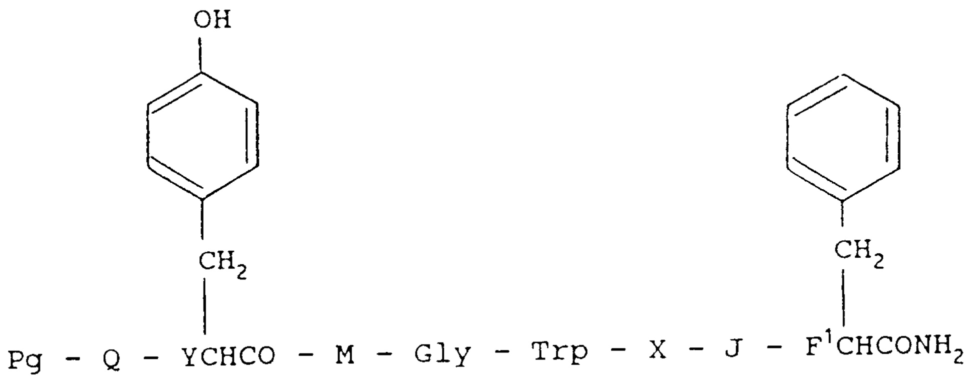

worin Pg eine geeignete Schutzgruppe ist und Q, Y, M, X, J und $F^1$ wie in Anspruch 1 definiert sind, welches sulfatiert und entschützt wird.

**Patentansprüche für folgende Vertragsstaaten : AT, ES, GR,**

**1.** Verfahren zur Herstellung eines Peptids der Formel

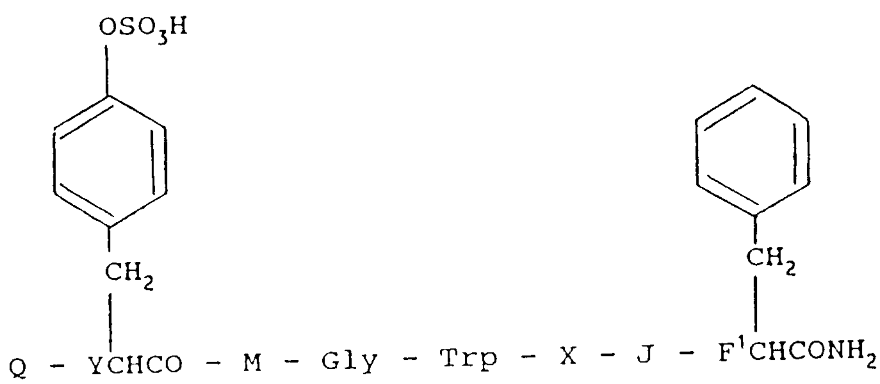

worin
Q H-$\beta$Asp, H-DAsp, For, Ac, Suc, desQ oder $R^1R^2$CHOCO ist;
Y H, (S)-NH oder (S)-$R^3$N ist;
M Met, Ahx, Leu oder Ile ist;
X Met, Ahx, Leu oder Ile ist;
J Asp, DAsp oder MeAsp ist;
$F^1$ (S)-NH oder (S)-$R^4$N ist;
$R^1$ und $R^2$ unabhängig voneinander H oder nied.Alkyl darstellen;
$R^3$ und $R^4$ nied.Alkyl darstellen;
und pharmazeutisch akzeptabler Salze davon;
mit der Maßgabe, daß:
(1) Q desQ ist, wenn Y H ist;
(2) Q nicht Ac ist, wenn im selben Peptid: Y (S)-NH ist; M Met, Ahx oder Leu ist; X Met, Ahx oder Leu ist; J Asp ist; und $F^1$ (S)-NH ist;
(3) Q weder H-$\beta$Asp noch For ist, wenn im selben Peptid: Y (S)-NH ist; M Met, Ahx oder Leu ist; X Met, Ahx oder Leu ist; J Asp ist; und $F^1$ (S)-NH ist;
(4) Y nicht H ist, wenn im selben Peptid: M Met ist; X Met ist; J Asp ist; und $F^1$ (S)-NH ist;
(5) Q nicht Suc ist, wenn im selben Peptid: Y (S)-NH ist; M Met ist; X Met ist; und J Asp ist; und
(6) Q nicht H-DAsp ist, wenn im selben Peptid: Y (S)-NH ist; M Met ist; X Met ist; J Asp ist; und $F^1$ (S)-NH ist;

mittels der Feststoffphasenmethode durch sequentielle Zugabe (jeweils mit Entschützung im Anschluß an jede Zugabe) der erforderlichen geschützten Aminosäure oder des erforderlichen geschützten Oligopeptids zu einem Aminoacylharz der Formel

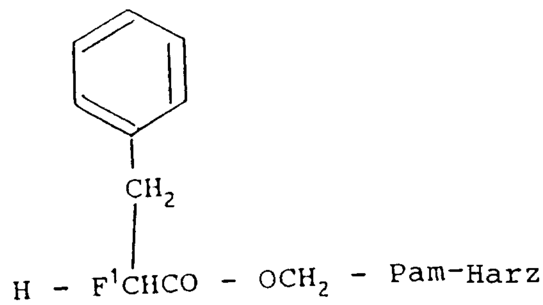

worin $F^1$ wie oben definiert ist,
zur Lieferung eines geschützten Peptidylharzes der Formel

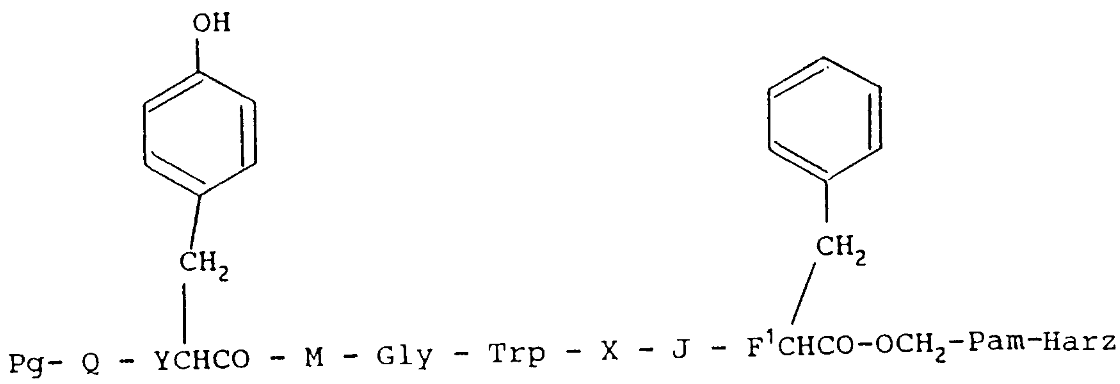

worin Pg eine geeignete Schutzgruppe ist und Q, Y, M, X, J und $F^1$ wie oben definiert sind, welches sulfatiert, entschützt und mit einem Methanolamin behandelt wird.

**2.** Verfahren zur Herstellung eines Peptids der Formel

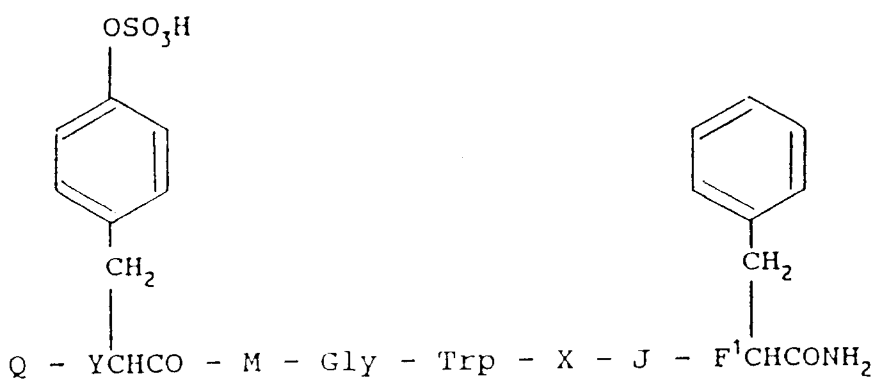

worin
Q H-$\beta$Asp, H-DAsp, For, Ac, Suc, desQ oder $R^1R^2CHOCO$ ist;
Y H, (S)-NH oder (S)-$R^3N$ ist;
M Met, Ahx, Leu oder Ile ist;
X Met, Ahx, Leu oder Ile ist;
J Asp, DAsp oder MeAsp ist;
$F^1$ (S)-NH oder (S)-$R^4N$ ist;
$R^1$ und $R^2$ unabhängig voneinander H oder nied.Alkyl darstellen;
$R^3$ und $R^4$ nied.Alkyl darstellen;
und pharmazeutisch akzeptabler Salze davon;
mit der Maßgabe, daß:
(1) Q desQ ist, wenn Y H ist;

(2) Q nicht Ac ist, wenn im selben Peptid: Y (S)-NH ist; M Met, Ahx oder Leu ist; X Met, Ahx oder Leu ist; J Asp ist; und $F^1$ (S)-NH ist;

(3) Q weder H-βAsp noch For ist, wenn im selben Peptid: Y (S)-NH ist; M Met, Ahx oder Leu ist; X Met, Ahx oder Leu ist; J Asp ist; und $F^1$ (S)-NH ist;

(4) Y nicht H ist, wenn im selben Peptid: M Met ist; X Met ist; J Asp ist; und $F^1$ (S)-NH ist;

(5) Q nicht Suc ist, wenn im selben Peptid: Y (S)-NH ist; M Met ist; X Met ist; und J Asp ist; und

(6) Q nicht H-DAsp ist, wenn im selben Peptid: Y (S)-NH ist; M Met ist; X Met ist; J Asp ist; und $F^1$ (S)-NH ist;

mittels der Lösungsphasenmethode durch sequentielle Zugabe (jeweils mit Entschützung im Anschluß an jede Zugabe) der erforderlichen geschützten Aminosäure oder des erforderlichen geschützten Oligopeptids zu einem Aminoacylamid der Formel

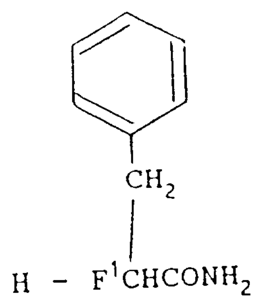

worin $F^1$ wie oben definiert ist,
zur Lieferung eines geschützten Peptidylamids der Formel

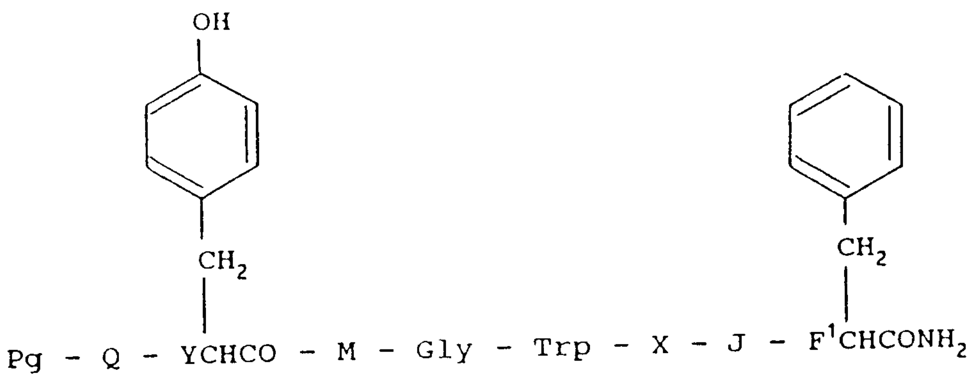

worin Pg eine geeignete Schutzgruppe ist und Q, Y, M, X, J und $F^1$ wie oben definiert sind, welches sulfatiert und entschützt wird.

**3.** Verfahren nach Anspruch 1 oder 2, worin das hergestellte Peptid iBuOCO-Tyr($SO_3H$)-Met-Gly-Trp-Met-Asp-Phe-$NH_2$ oder ein pharmazeutisch akzeptables Salz davon ist.

**4.** Verfahren nach Anspruch 1 oder 2, worin das hergestellte Peptid Suc-Tyr($SO_3H$)-Ahx-Gly-Trp-Ahx-Asp-Phe-$NHC_2$ oder ein pharmazeutisch akzeptables Salz davon ist.

**5.** Verfahren nach Anspruch 1 oder 2, worin das hergestellte Peptid H-βAsp-Tyr($SO_3H$)-Met-Gly-Trp-Met-Asp-MePhe-$NH_2$ oder ein pharmazeutisch akzeptables Salz davon ist.

**6.** Verfahren nach Anspruch 1 oder 2, worin das hergestellte Peptid H-DAsp-Tyr($SO_3H$)-Met-Gly-Trp-Met-Asp-MePhe-$NH_2$ oder ein pharmazeutisch akzeptables Salz davon ist.

**7.** Verfahren nach Anspruch 1 oder 2, worin das hergestellte Peptid For-Tyr($SO_3H$)-Met-Gly-Trp-Met-Asp-MePhe-$NH_2$ oder ein pharmazeutisch akzeptables Salz davon ist.

8. Verfahren nach Anspruch 1 oder 2, worin das hergestellte Peptid iBuOCO-Tyr($SO_3H$)-Met-Gly-Trp-Met-Asp-MePhe-$NH_2$ oder ein pharmazeutisch akzeptables Salz davon ist.

9. Verfahren nach Anspruch 1 oder 2, worin das hergestellte Peptid Hpp($SO_3H$)-Met-Gly-Trp-Met-Asp-MePhe-$NH_2$ oder ein pharmazeutisch akzeptables Salz davon ist.

10. Verfahren nach Anspruch 1 oder 2, worin das hergestellte Peptid PrOCO-Tyr($SO_3H$)-Met-Gly-Trp-Met-Asp-Phe-$NH_2$ oder ein pharmazeutisch akzeptables Salz davon ist.

11. Verfahren nach Anspruch 1 oder 2, worin das hergestellte Peptid EtOCO-Tyr($SO_3H$)-Met-Gly-Trp-Met-Asp-Phe-$NH_2$ oder ein pharmazeutisch akzeptables Salz davon ist.

12. Verfahren nach Anspruch 1 oder 2, worin das hergestellte Peptid MeOCO-Tyr($SO_3H$)-Met-Gly-Trp-Met-Asp-Phe-$NH_2$ oder ein pharmazeutisch akzeptables Salz davon ist.

13. Verfahren nach Anspruch 1 oder 2, worin das hergestellte Peptid Suc-Tyr($SO_3H$)-Ahx-Gly-Trp-Ahx-Asp-MePhe-$NH_2$ oder ein pharmazeutisch akzeptables Salz davon ist.

14. Verfahren nach Anspruch 1 oder 2, worin das hergestellte Peptid iBuOCO-Tyr($SO_3H$)-Ahx-Gly-Trp-Ahx-Asp-MePhe-$NH_2$ oder ein pharmazeutisch akzeptables Salz davon ist.

15. Verfahren nach Anspruch 1 oder 2, worin das hergestellte Peptid Hpp($SO_3H$)-Ahx-Gly-Trp-Ahx-Asp-MePhe-$NH_2$ oder ein pharmazeutisch akzeptables Salz davon ist.

16. Verfahren nach Anspruch 1 oder 2, worin das hergestellte Peptid iBuOCO-Tyr($SO_3H$)-Ahx-Gly-Trp-Ahx-Asp-Phe-$NH_2$ oder ein pharmazeutisch akzeptables Salz davon ist.

17. Verfahren nach Anspruch 1 oder 2, worin das hergestellte Peptid Hpp($SO_3H$)-Ahx-Gly-Trp-Ahx-Asp-Phe-$NH_2$ oder ein pharmazeutisch akzeptables Salz davon ist.

18. Verfahren nach Anspruch 1 oder 2, worin das hergestellte Peptid For-Tyr($SO_3H$)-Ile-Gly-Trp-Ile-Asp-Phe-$NH_2$ oder ein pharmazeutisch akzeptables Salz davon ist.

19. Verfahren nach Anspruch 1 oder 2, worin das hergestellte Peptid Suc-Tyr($SO_3H$)-Ile-Gly-Trp-Ile-Asp-Phe-$NH_2$ oder ein pharmazeutisch akzeptables Salz davon ist.

20. Verfahren nach Anspruch 1 oder 2, worin das hergestellte Peptid iBuOCO-Tyr($SO_3H$)-Ile-Gly-Trp-Ile-Asp-Phe-$NH_2$ oder ein pharmazeutisch akzeptables Salz davon ist.

21. Verfahren nach Anspruch 1 oder 2, worin das hergestellte Peptid Hpp($SO_3H$)-Ile-Gly-Trp-Ile-Asp-Phe-$NH_2$ oder ein pharmazeutisch akzeptables Salz davon ist.

22. Verfahren zur Verbesserung der körperlichen Erscheinung eines Menschen, welches die intraperitoneale, intravenöse, intramuskuläre, subkutane oder intranasale Verabreichung eines Peptids nach Anspruch 1, aber ohne den Maßgaben (2) - (6), oder eines pharmazeutisch akzeptablen Salzes davon an diesen Menschen bis zum Eintreten eines kosmetisch günstigen Verlusts an Körpergewicht umfaßt.

**Revendications**

**Revendications pour les Etats contractants suivants : BE, CH, LI, DE, FR, GB, IT, LU, NL, SE**

1. Peptide de formule :

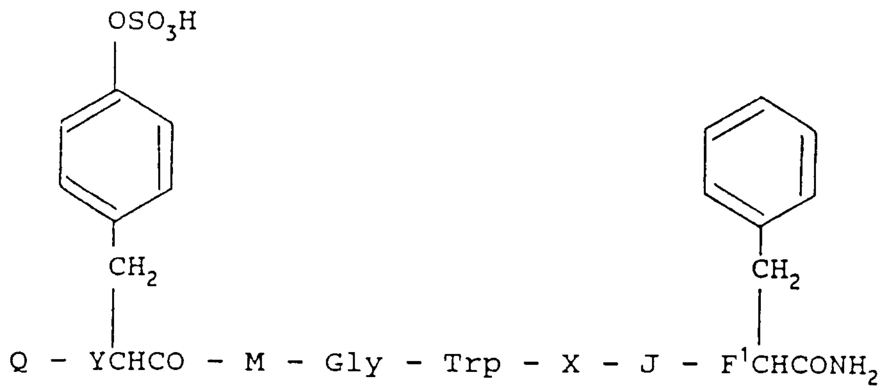

où
Q est H-$\beta$Asp, H-DAsp, For, Ac, Suc, desQ ou $R^1R^2CHOCO$;
Y est H, (S)-HN ou (S)-$R^3N$;
M est Met, Ahx, Leu ou Ile;
X est Met, Ahx, Leu ou Ile;
J est Asp, DAsp ou MeAsp;
$F^1$ est (S)-NH ou (S)-$R^4N$;
$R^1$ et $R^2$ représentent indépendamment H ou un alcoyle inférieur;
$R^3$ et $R^4$ représentent un alcoyle inférieur;
et un sel pharmaceutiquement acceptable de celui-ci;
à la condition que :
(1) Q est desQ quand Y est H;
(2) Q n'est pas Ac si, dans le même peptide : Y est (S)-NH; M est Met, Ahx ou Leu; X est Met, Ahx ou Leu; J est Asp; et $F^1$ est (S)-NH;
(3) Q n'est ni H-$\beta$Asp ni For si, dans le même peptide : Y est (S)-NH; M est Met, Ahx ou Leu; X est Met, Ahx ou Leu; J est Asp; et $F^1$ est (S)-NH;
(4) Y n'est pas H si, dans le même peptide : M est Met; X est Met; J est Asp; et $F^1$ est (S)-NH;
(5) Q n'est pas Suc si, dans le même peptide : Y est (S)-NH; M est Met; X est Met; et J est Asp, et
(6) Q n'est pas H-DAsp si, dans le même peptide : Y est (S)-NH; M est Met; X est Met; J est Asp; et $F^1$ est (S)-NH.

2. Peptide suivant la revendication 1, qui est
iBuOCO-Tyr($SO_3H$)-Met-Gly-Trp-Met-Asp-Phe-$NH_2$,
ou un sel pharmaceutiquement acceptable de celui-ci.

3. Peptide suivant la revendication 1, qui est
Suc-Tyr($SO_3H$)-Ahx-Gly-Trp-Ahx-Asp-Phe-$NH_2$,
ou un sel pharmaceutiquement acceptable de celui-ci.

4. Peptide suivant la revendication 1, qui est
H-$\beta$Asp-Tyr($SO_3H$)-Met-Gly-Trp-Met-Asp-MePhe-$NH_2$,
ou un sel pharmaceutiquement acceptable de celui-ci.

5. Peptide suivant la revendication 1, qui est
H-DAsp-Tyr($SO_3H$)-Met-Gly-Trp-Met-Asp-MePhe-$NH_2$,
ou un sel pharmaceutiquement acceptable de celui-ci.

**6.** Peptide suivant la revendication 1, qui est
For-Tyr($SO_3H$)-Met-Gly-Trp-Met-Asp-MePhe-$NH_2$,
ou un sel pharmaceutiquement acceptable de celui-ci.

**7.** Peptide suivant la revendication 1, qui est
iBuOCO-Tyr($SO_3H$)-Met-Gly-Trp-Met-Asp-MePhe-$NH_2$,
ou un sel pharmaceutiquement acceptable de celui-ci.

**8.** Peptide suivant la revendication 1, qui est
Hpp($SO_3H$)-Met-Gly-Trp-Met-Asp-MePhe-$NH_2$,
ou un sel pharmaceutiquement acceptable de celui-ci.

**9.** Peptide suivant la revendication 1, qui est
PrOCO-Tyr($SO_3H$)-Met-Gly-Trp-Met-Asp-Phe-$NH_2$,
ou un sel pharmaceutiquement acceptable de celui-ci.

**10.** Peptide suivant la revendication 1, qui est
EtOCO-Tyr($SO_3H$)-Met-Gly-Trp-Met-Asp-Phe-$NH_2$,
ou un sel pharmaceutiquement acceptable de celui-ci.

**11.** Peptide suivant la revendication 1, qui est
MeOCO-Tyr($SO_3H$)-Met-Gly-Trp-Met-Asp-Phe-$NH_2$,
ou un sel pharmaceutiquement acceptable de celui-ci.

**12.** Peptide suivant la revendication 1, qui est
Suc-Tyr($SO_3H$)-Ahx-Gly-Trp-Ahx-Asp-MePhe-$NH_2$,
ou un sel pharmaceutiquement acceptable de celui-ci.

**13.** Peptide suivant la revendication 1, qui est
iBuOCO-Tyr($SO_3H$)-Ahx-Gly-Trp-Ahx-Asp-MePhe-$NH_2$,
ou un sel pharmaceutiquement acceptable de celui-ci.

**14.** Peptide suivant la revendication 1, qui est
Hpp($SO_3H$)-Ahx-Gly-Trp-Ahx-Asp-MePhe-$NH_2$,
ou un sel pharmaceutiquement acceptable de celui-ci.

**15.** Peptide suivant la revendication 1, qui est
iBuOCO-Tyr($SO_3H$)-Ahx-Gly-Trp-Ahx-Asp-Phe-$NH_2$,
ou un sel pharmaceutiquement acceptable de celui-ci.

**16.** Peptide suivant la revendication 1, qui est
Hpp($SO_3H$)-Ahx-Gly-Trp-Ahx-Asp-Phe-$NH_2$,
ou un sel pharmaceutiquement acceptable de celui-ci.

**17.** Peptide suivant la revendication 1, qui est
For-Tyr($SO_3H$)-Ile-Gly-Trp-Ile-Asp-Phe-$NH_2$,
ou un sel pharmaceutiquement acceptable de celui-ci.

**18.** Peptide suivant la revendication 1, qui est
Suc-Tyr($SO_3H$)-Ile-Gly-Trp-Ile-Asp-Phe-$NH_2$,
ou un sel pharmaceutiquement acceptable de celui-ci.

**19.** Peptide suivant la revendication 1, qui est
iBuOCO-Tyr($SO_3H$)-Ile-Gly-Trp-Ile-Asp-Phe-$NH_2$,
ou un sel pharmaceutiquement acceptable de celui-ci.

**20.** Peptide suivant la revendication 1, qui est
Hpp($SO_3H$)-Ile-Gly-Trp-Ile-Asp-Phe-$NH_2$,

ou un sel pharmaceutiquement acceptable de celui-ci.

**21.** Composition pour la prévention de l'obésité, comprenant un peptide tel que défini dans la revendication 1, mais sans les conditions (2) à (6).

**22.** Utilisation d'un peptide tel que défini dans la revendication 1, comme produit pharmaceutique.

**23.** Utilisation d'un peptide tel que défini dans la revendication 1, mais sans les conditions (2) à (6), dans la fabrication d'un médicament pour le traitement de l'obésité.

**24.** Procédé pour améliorer l'aspect physique d'un être humain, qui comprend l'administration à un être humain de manière intrapéritonéale, intraveineuse, intramusculaire, sous-cutanée ou intranasale, d'un peptide tel que défini dans la revendication 1, mais sans les conditions (2) à (6), ou un sel pharmaceutiquement acceptable de celui-ci, jusqu'à ce qu'une perte cosmétiquement bénéfique du poids du corps se produise.

**25.** Procédé pour la production d'un peptide tel que défini dans la revendication 1, par la méthode en phase solide d'addition séquentielle (suivie par déprotection après chaque addition) de l'acide aminé protégé requis ou de l'oligopeptide protégé requis à une résine aminoacyle de formule :

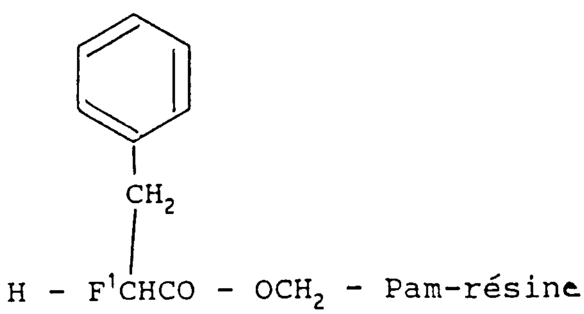

où $F^1$ est tel que défini dans la revendication 1, pour obtenir une résine peptidyle protégée de formule :

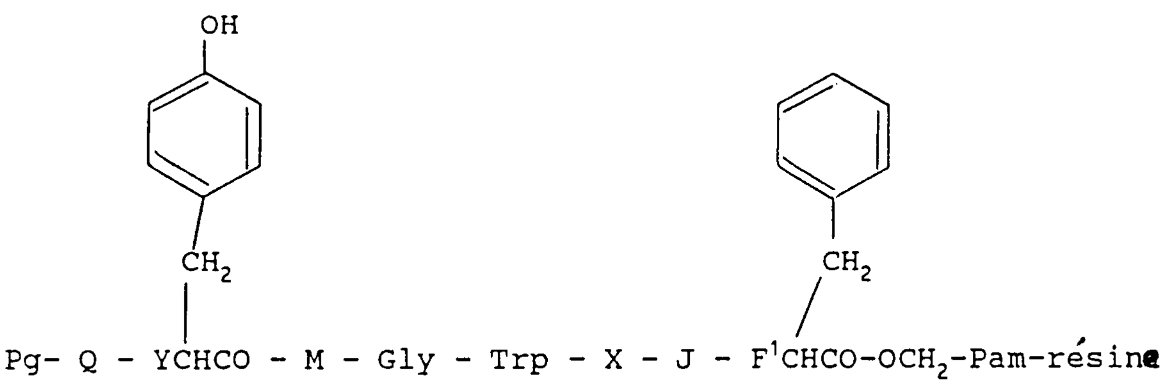

où Pg est un groupe protecteur approprié et Q, Y, M, X, J et $F^1$ sont tels que définis dans la revendication 1, qui est sulfatée, déprotégée et traitée avec une amine méthanolique.

**26.** Procédé pour la protection d'un peptide tel que défini dans la revendication 1, par le procédé en phase liquide d'addition séquentielle (suivie par déprotection après chaque addition) de l'acide aminé protégé requis ou de l'oligopeptide protégé requis à un amide aminoacyle de formule :

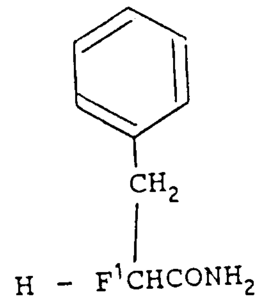

où $F^1$ est tel que défini dans la revendication 1, pour obtenir un amide peptidique protégé de formule :

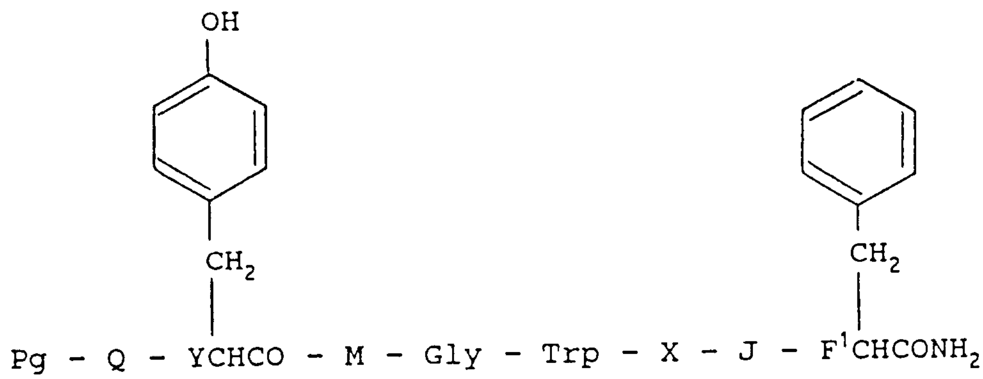

où Pg est un groupe protecteur approprié et Q, Y, M, X, J et $F^1$ sont tels que définis dans la revendication 1, qui est sulfaté et déprotégé.

**Revendications pour les Etats contractants suivants : AT, ES, GR**

**1.** Procédé pour la préparation d'un peptide de formule :

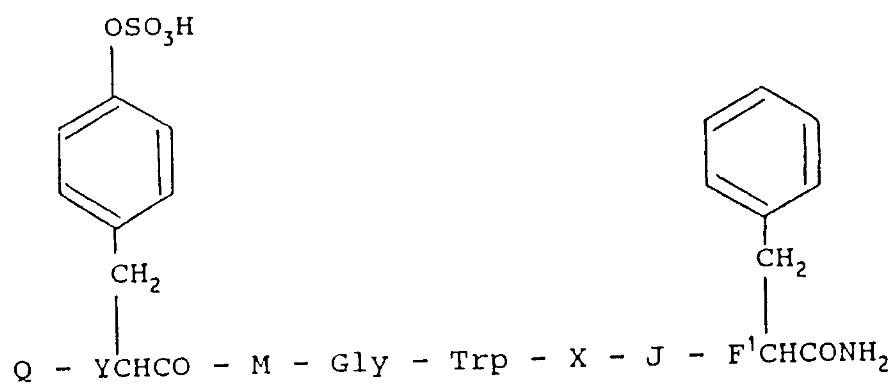

où
Q est H-$\beta$Asp, H-DAsp, For, Ac, Suc, desQ ou $R^1R^2$CHOCO;
Y est H, (S)-HN ou (S)-$R^3$N;
M est Met, Ahx, Leu ou Ile;
X est Met, Ahx, Leu ou Ile;
J est Asp, DAsp ou MeAsp;
$F^1$ est (S)-NH ou (S)-$R^4$N;
$R^1$ et $R^2$ représentent indépendamment H ou un alcoyle inférieur;
$R^3$ et $R^4$ représentent un alcoyle inférieur;
et un sel pharmaceutiquement acceptable de celui-ci;

à la condition que :

(1) Q est desQ quand Y est H;

(2) Q n'est pas Ac si, dans le même peptide : Y est (S)-NH; M est Met, Ahx ou Leu; X est Met, Ahx ou Leu; J est Asp; et $F^1$ est (S)-NH;

(3) Q n'est ni H-$\beta$Asp ni For si, dans le même peptide : Y est (S)-NH; M est Met, Ahx ou Leu; X est Met, Ahx ou Leu; J est Asp; et $F^1$ est (S)-NH;

(4) Y n'est pas H si, dans le même peptide : M est Met; X est Met; J est Asp; et $F^1$ est (S)-NH;

(5) Q n'est pas Suc si, dans le même peptide : Y est (S)-NH; M est Met; X est Met; et J est Asp, et

(6) Q n'est pas H-DAsp si, dans le même peptide : Y est (S)-NH; M est Met; X est Met; J est Asp; et $F^1$ est (S)-NH

par la méthode en phase solide d'addition séquentielle (suivie par déprotection après chaque addition) de l'acide aminé protégé requis ou de l'oligopeptide protégé requis à une résine aminoacyle de formule :

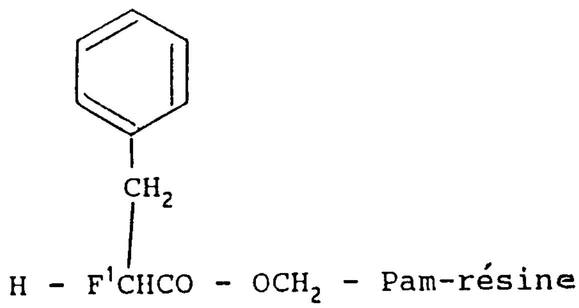

où $F^1$ est tel que défini ci-dessus, pour obtenir une résine peptidyle protégée de formule :

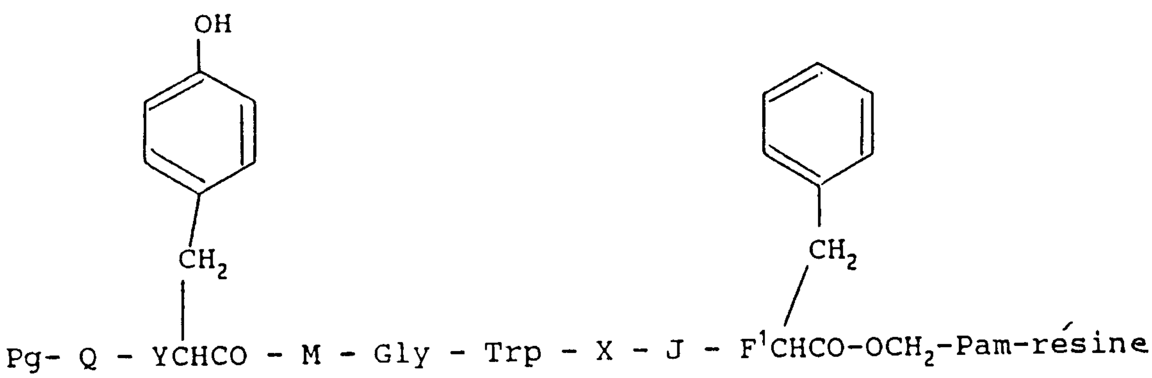

où Pg est un groupe protecteur approprié et Q, Y, M, X, J et $F^1$ sont tels que définis ci-dessus, qui est sulfatée, déprotégée et traitée avec une amine méthanolique.

**2.** Procédé pour la préparation d'un peptide de formule :

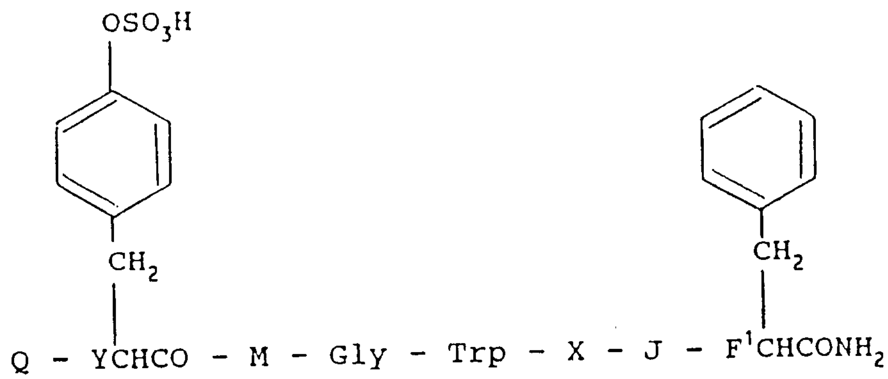

où
Q est H-βAsp, H-DAsp, For, Ac, Suc, desQ ou $R^1R^2CHOCO$;
Y est H, (S)-HN ou (S)-$R^3N$;
M est Met, Ahx, Leu ou Ile;
X est Met, Ahx, Leu ou Ile;
J est Asp, DAsp ou MeAsp;
$F^1$ est (S)-NH ou (S)-$R^4N$;
$R^1$ et $R^2$ représentent indépendamment H ou un alcoyle inférieur;
$R^3$ et $R^4$ représentent un alcoyle inférieur;
et un sel pharmaceutiquement acceptable de celui-ci;
à la condition que :

(1) Q est desQ quand Y est H;
(2) Q n'est pas Ac si, dans le même peptide : Y est (S)-NH; M est Met, Ahx ou Leu; X est Met, Ahx ou Leu; J est Asp; et $F^1$ est (S)-NH;
(3) Q n'est ni H-βAsp ni For si, dans le même peptide : Y est (S)-NH; M est Met, Ahx ou Leu; X est Met, Ahx ou Leu; J est Asp; et $F^1$ est (S)-NH;
(4) Y n'est pas H si, dans le même peptide : M est Met; X est Met; J est Asp; et $F^1$ est (S)-NH;
(5) Q n'est pas Suc si, dans le même peptide : Y est (S)-NH; M est Met; X est Met; et J est Asp, et
(6) Q n'est pas H-DAsp si, dans le même peptide : Y est (S)-NH; M est Met; X est Met; J est Asp; et $F^1$ est (S)-NH

par le procédé en phase liquide d'addition séquentielle (suivie par déprotection après chaque addition) de l'acide aminé protégé requis ou de l'oligopeptide protégé requis à un amide aminoacyle de formule :

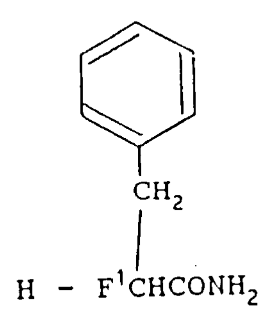

où $F^1$ est tel que défini ci-dessus, pour obtenir un amide peptidique protégé de formule :

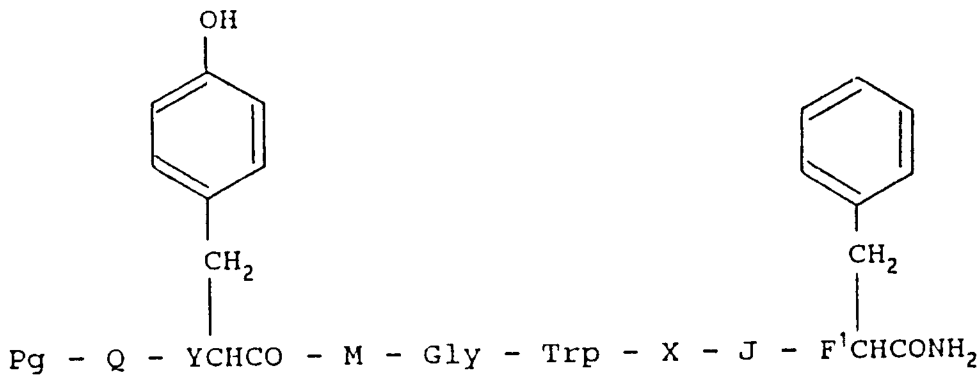

où Pg est un groupe protecteur approprié et Q, Y, M, X, J et $F^1$ sont tels que définis ci-dessus, qui est sulfaté et déprotégé.

**3.** Procédé suivant la revendication 1 ou la revendication 2, où le peptide préparé est
iBuOCO-Tyr($SO_3H$)-Met-Gly-Trp-Met-Asp-Phe-$NH_2$,
ou un sel pharmaceutiquement acceptable de celui-ci.

**4.** Procédé suivant la revendication 1 ou la revendication 2, où le peptide préparé est
Suc-Tyr($SO_3H$)-Ahx-Gly-Trp-Ahx-Asp-Phe-$NH_2$,
ou un sel pharmaceutiquement acceptable de celui-ci.

**5.** Procédé suivant la revendication 1 ou la revendication 2, qui est
H-$\beta$Asp-Tyr($SO_3H$)-Met-Gly-Trp-Met-Asp-MePhe-$NH_2$,
ou un sel pharmaceutiquement acceptable de celui-ci.

**6.** Procédé suivant la revendication 1 ou la revendication 21, qui est
H-DAsp-Tyr($SO_3H$)-Met-Gly-Trp-Met-Asp-MePhe-$NH_2$,
ou un sel pharmaceutiquement acceptable de celui-ci.

**7.** Procédé suivant la revendication 1 ou la revendication 2, qui est
For-Tyr($SO_3H$)-Met-Gly-Trp-Met-Asp-MePhe-$NH_2$,
ou un sel pharmaceutiquement acceptable de celui-ci.

**8.** Procédé suivant la revendication 1 ou la revendication 2, qui est
iBuOCO-Tyr($SO_3H$)-Met-Gly-Trp-Met-Asp-MePhe-$NH_2$,
ou un sel pharmaceutiquement acceptable de celui-ci.

**9.** Procédé suivant la revendication 1 ou la revendication 2, qui est
Hpp($SO_3H$)-Met-Gly-Trp-Met-Asp-MePhe-$NH_2$,
ou un sel pharmaceutiquement acceptable de celui-ci.

**10.** Procédé suivant la revendication 1 ou la revendication 2, qui est
PrOCO-Tyr($SO_3H$)-Met-Gly-Trp-Met-Asp-Phe-$NH_2$,
ou un sel pharmaceutiquement acceptable de celui-ci.

**11.** Procédé suivant la revendication 1 ou la revendication 2, qui est
EtOCO-Tyr($SO_3H$)-Met-Gly-Trp-Met-Asp-Phe-$NH_2$,
ou un sel pharmaceutiquement acceptable de celui-ci.

**12.** Procédé suivant la revendication 1 ou la revendication 2, qui est
MeOCO-Tyr($SO_3H$)-Met-Gly-Trp-Met-Asp-Phe-$NH_2$,
ou un sel pharmaceutiquement acceptable de celui-ci.

13. Procédé suivant la revendication 1 ou la revendication 2, qui est
Suc-Tyr($SO_3H$)-Ahx-Gly-Trp-Ahx-Asp-MePhe-$NH_2$,
ou un sel pharmaceutiquement acceptable de celui-ci

14. Peptide suivant la revendication 1 ou la revendication 2, qui est
iBuOCO-Tyr($SO_3H$)-Ahx-Gly-Trp-Ahx-Asp-MePhe-$NH_2$,
ou un sel pharmaceutiquement acceptable de celui-ci.

15. Procédé suivant la revendication 1 ou la revendication 2, qui est
Hpp($SO_3H$)-Ahx-Gly-Trp-Ahx-Asp-MePhe-$NH_2$,
ou un sel pharmaceutiquement acceptable de celui-ci.

16. Procédé suivant la revendication 1 ou la revendication 2, qui est
iBuOCO-Tyr($SO_3H$)-Ahx-Gly-Trp-Ahx-Asp-Phe-$NH_2$,
ou un sel pharmaceutiquement acceptable de celui-ci.

17. Procédé suivant la revendication 1 ou la revendication 2, qui est
Hpp($SO_3H$)-Ahx-Gly-Trp-Ahx-Asp-Phe-$NH_2$,
ou un sel pharmaceutiquement acceptable de celui-ci.

18. Procédé suivant la revendication 1 ou la revendication 2, qui est
For-Tyr($SO_3H$)-Ile-Gly-Trp-Ile-Asp-Phe-$NH_2$,
ou un sel pharmaceutiquement acceptable de celui-ci.

19. Procédé suivant la revendication 1 ou la revendication 2, qui est
Suc-Tyr($SO_3H$)-Ile-Gly-Trp-Ile-Asp-Phe-$NH_2$,
ou un sel pharmaceutiquement acceptable de celui-ci.

20. Procédé suivant la revendication 1 ou la revendication 2, qui est
iBuOCO-Tyr($SO_3H$)-Ile-Gly-Trp-Ile-Asp-Phe-$NH_2$,
ou un sel pharmaceutiquement acceptable de celui-ci.

21. Procédé suivant la revendication 1 ou la revendication 2, qui est
Hpp($SO_3H$)-Ile-Gly-Trp-Ile-Asp-Phe-$NH_2$,
ou un sel pharmaceutiquement acceptable de celui-ci.

22. Procédé pour améliorer l'aspect physique d'un être humain qui comprend l'administration à un être humain, de manière intrapéritonéale, intraveineuse, intramusculaire, sous-cutanée ou intranasale, d'un peptide tel que défini dans la revendication 1, mais sans les conditions (2) à (6), ou d'un sel pharmaceutiquement acceptable de celui-ci, jusqu'à ce qu'une perte cosmétiquement bénéfique du poids du corps soit obtenue.